# EUROPEAN PATENT SPECIFICATION

(11) **EP 3 528 858 B1**
(45) Date of publication and mention of the grant of the patent: **21.10.2020**
(21) Application number: 17794904.7
(22) Date of filing: 17.10.2017
(51) Int. Cl.: A61L 27/20, A61L 27/52, A61L 27/56, A61L 27/58, A61L 15/28, A61L 15/42, A61L 15/60

(54) **PRINTED HYALURONIC ACID SCAFFOLDS**
GEDRUCKTE HYALURONSÄUREGERÜSTE
ÉCHAFAUDAGES D'ACIDE HYALURONIQUE IMPRIMÉS

(30) Priority: 18.10.2016 DK PA201670820
(43) Date of publication of application: 28.08.2019
(73) Proprietor: Aarhus Universitet, 8000 Aarhus C (DK)
(72) Inventor: LE, Dang, Quang, Svend, 8250 Egå (DK); CHEN, Muwan, 8250 Egå (DK)
(74) Representative: Aera A/S
(86) International application number: PCT/EP2017/076453
(87) International publication number: WO 2018/073235

(56) References cited:
- EP-A1- 2 679 669
- WO-A1-2009/047347
- WO-A1-2014/206500
- WO-A2-2009/108760
- WO-A2-2010/029344
- WO-A2-2010/115081
- LILIANG OUYANG ET AL: "3D Printing of Shear-Thinning Hyaluronic Acid Hydrogels with Secondary Cross-Linking", ACS BIOMATERIALS SCIENCE & ENGINEERING, vol. 2, no. 10, 9 June 2016 (2016-06-09), pages 1743-1751, XP055440741, ISSN: 2373-9878, DOI: 10.1021/acsbiomaterials.6b00158

## Description

### Technical field

The present invention relates a hydrogel comprising a porous structure of ordered structural elements, wherein said structural elements comprises crosslinked hyaluronic acid. The present invention also relates to the use of said hydrogels and methods for preparing said hydrogels.

### Background

Biocompatible three dimensional hydrogels are highly valued tools in biotechnology, biomedicine, biomedical engineering, regenerative medicine, drug discovery and tissue engineering.

Hyaluronic acid (HA) is a natural and linear carbohydrate polymer belonging to the class of non-sulfated glucosaminoglycans. HA is present in the majority of tissues where it forms a crucial part of the extracellular matrix and tissue integrity. HA is part of essential processes such as joint lubrication, tissue water content and turgor. HA has traditionally been sourced from animal sources e.g. rooster comb and bovine vitrous humor. As bacterial fermentation methods have been developed and validated, HA presents itself as a very versatile, tunable, animal-free biomaterial for fabrication of scaffolds. HA dissolves in water and forms a clear colorless liquid with viscosity dependent on HA concentration and molecular weight.

HA readily forms hydrogels with appropriate crosslinking, which can be achieved with crosslinking agents such as for example divinyl sulfone (DVS), carbodiimide or adipic acid dehydrate. Divinyl sulfone (DVS) crosslinked Hyaluronic acid hydrogels have an excellent clinical track record as FDA approved class III medical devices.

During recent years Three-dimensional (3D) hydrogels and cell culture systems have gained increasing interest in biomedical engineering, regenerative medicine, drug discovery and tissue engineering due to their advantages in providing more physiologically relevant information than 2D cell culture systems.

3D hydrogels should preferentially comprise an interconnected porous network to improve metabolite transport and cell ingrowth. To create 3D hydrogels, several methods are available such as casting, electrospinning and thermally induced phase separation. These methods provide a bottom up approach to create a microstructured network. However, to create 3D hydrogels with a user defined shape, macro- and microstructure, additive manufacturing methods are advantageous. The ISO/ASTM 52900 standard divides additive manufacturing into seven categories: 1) VAT Photopolymerisation 2) Binder Jetting 3) Material Jetting 4) Material Extrusion 5) Powder Bed Fusion 6) Sheet Lamination 7) Directed Energy Deposition. The fourth category, Material extrusion, which comprises the fused deposition modelling method, employ a special printer or bioprinter is used that may create 3D constructs in a layer-by-layer method. However, the success of printing biocompatible 3D hydrogels so far has been limited by a lack of biomaterials that are compatible with printing devices and having the optimal balance of robustness, extrudability, biocompatibility, and biodegradability. Thus, it has been a challenge to obtain biomaterials that are easy to handle and extrude such that the desired geometry of the scaffold or hydrogel is obtaind and at the same time result in hydrogels having the required stability and homogeneity.

LILIANG OUYANG ET AL: "3D Printing of Shear-Thinning Hyaluronic Acid Hydrogels with Secondary Cross-Linking",ACS BIOMATERIALS SCIENCE & ENGINEERING, vol. 2, no. 10, 9 June 2016 (2016-06-09), pages 1743-1751, XP055440741, discloses 3D-printed filamentous biomaterial comprising dual-crosslinked chemically modified hyaluronic acid. WO2010029344 discloses porous hyaluronic acid structures for biomedical and cosmetic applications. WO2010115081 discloses methods of processing hydrogels comprising crosslinked hyaluronic acid into products of various shape. WO2013055832 discloses threads comprising BDDE-crosslinked hyaluronic acid and three-dimensional structures constructed with them. EP3235470/WO2016097448 disclose tubular hybrid scaffolds, comprising porous hydrogel based on cross-linked hyaluronic acid.

### Summary

The invention is defined by the claims. Any subject matter falling outside the scope of the claims is provided for information purposes only. Provided are crosslinked hyaluronic acid hydrogel scaffolds with a well-defined homogeneous structure and porous network. The hydrogels are biocompatible and preferably have a 3D structure. The invention also provides methods for creating said Hyaluronic acid hydrogels that are easy to handle such that the predetermined geometry of the hydrogel can be obtained and at the same time results in a stable hydrogel scaffold having a homogeneous pore network. Accordingly, one aspect of the present invention relates to a hydrogel comprising a porous structure of ordered structural elements, wherein said structural elements comprises crosslinked hyaluronic acid.

Said hydrogel comprises multiple layers.

The structural elements preferably form a hierarchical structure. In one embodiment the hydrogel comprises or consists of a hierarchical structure.

It is preferred that said porous structure comprises interconnected pores. For example, said pores have a width of from 0.05 mm to 5 mm. Preferably, said pores have a width of from 0.1 mm to 3 mm.

Said structural elements are fibres and/or particles.

Said fibres have a diameter in the range of from 50 µm to 1000 µm.

Preferably, said fibres have a diameter in the range of from 100 µm to 300 µm. Ina particular embodiment said fibres have a diameter of about 200 µm.

In one embodiment the hydrogel comprises a plurality of parallel fibres. In a preferred embodiment thereof, the hydrogel comprises a plurality of parallel fibres within the same layer. Said hydrogel comprises multiple layers wherein fibres between neighbouring layers are not parallel. The hydrogel can also comprise a plurality of non-intersecting fibres within the same layer.

In one embodiment said fibres are straight. In another embodiment said fibres are coiled. In one embodiment said hydrogel comprises straight fibres and coiled fibres. At least one layer consists of or comprises alternating coiled and straight fibres.

In one embodiment said coiled fibres forms a periodic curve.

In one embodiment the fibres between neighbouring layers form an angle of at least 20°.

In one embodiment the hydrogel comprises or consists of particles, wherein said particles have a diameter in the range of from 30 µm to 1 mm. Said particles are aligned in rows. In one embodiment said hydrogel comprises multiple layers and wherein each layer comprises multiple rows of particles. Preferably, said rows are parallel.

Said hyaluronic acid is crosslinked with DVS, BDDE and/or tyramine. In a more specific embodiment said hyaluronic acid is crosslinked with DVS and/or BDDE

It is preferred that the hydrogel of the present invention is biocompatible.

The hydrogel may further comprise at least one additional component, such as for example a biologically active agent. In one embodiment, the hydrogel further comprises at least one additional component selected from the group consisting of calcium phosphates, synthetic water-soluble polymers, synthetic water insoluble polymers, plant derived carbohydrates, microbially derived carbohydrates, animal carbohydrates, extracellular matrix compounds, chitosan and derivatives thereof, fatty acids, soaps, micellar particles, amino acids, peptides, polypeptides, proteins, glycoproteins, proteoglycans, ribonucleic acids, glass, silicates, and insoluble ceramics.

In one embodiment said hydrogel further comprises cells. In one embodiment said hydrogel comprises a coating. For example, said coating is fibronectin.

The present invention also provides methods for producing the hydrogels as described herein.

Thus, in one aspect the invention relates to a method of preparing a crosslinked HA hydrogel having a predetermined pattern of ordered structural elements, said method comprising the steps of
a. mixing HA, DVS and solvent to obtain a mixture of said DVS, HA and solvent wherein said solvent has a pH below 9;
b. depositing said solution onto a cooled substrate to obtain a frozen construct;
c. contacting (or immersing) said frozen construct, which has not yet been crosslinked, with an alkaline solution having a pH above 10 at a temperature below the freezing point of the construct to cross-link HA with DVS,
whereby a cross-linked hydrogel is obtained.

In one embodiment said HA and said DVS are mixed before addition of solvent. HA can for example be in the form of a powder.

Thus, one embodiment relates to a method of preparing a hydrogel comprising the steps of
a. mixing hyaluronic acid (HA) powder with divinyl sulfone (DVS) to obtain a mixture of DVS and HA;
b. adding a solvent to said mixture to obtain a solution comprising HA and DVS;
c. depositing said solution onto a substrate to obtain a construct;
d. contacting said construct with an alkaline solution to cross-link HA with DVS,
whereby a cross-linked hydrogel is obtained.

Preferably said construct and said hydrogel is three-dimensional

It is preferred that the alkaline solution comprises base at a concentration of at least 100 mM. Preferably, the salt is selected from the group consisting of NaCl, KCl and LiCI.

In a preferred embodiment said alkaline solution comprises base at a concentration of at least 100 mM. Preferably, said base is selected from the group consisting of NaOH, LiOH, KOH, Mg(OH)₂, Ca(OH)₂ Sr(OH)₂, Ba(OH)₂

In one embodiment the ratio of HA to DVS is in the range of from 10:1 to 2:1 by weight. In another embodiment the ratio of HA to DVS is in the range of from 5:1 to 3:1 by weight.

In a preferred embodiment said solvent comprises a buffer. Said solvent has a pH below 9. Preferably, said solvent has a pH below 8, such as a pH below 7. In another preferred embodiment said solvent comprises one or more salts, such as for example NACI, KCI and/or LiCI. For example, said solvent comprises sodium and/or potassium ions. In a particular embodiment said solvent comprises sodium and/or potassium ions in a concentration of at least 100 mM.

In one embodiment of the present invention said solution comprising HA and DVS is printed onto a cooled substrate. For example, said substrate is cooled to a temperature below 0 degrees Celsius (°C). In another embodiment said substrate is cooled to a temperature below -20°C. The substrate may for example be cooled using dry ice.

In one embodiment said printing solution and/or said alkaline solution comprises at least one additional component selected from the group consisting of calcium phosphates, synthetic watersoluble polymers, synthetic waterinsoluble polymers, plant derived carbohydrates, microbially derived carbohydrates, animal carbohydrates, extracellular matrix compounds, chitosan and derivatives thereof, fatty acids, soaps, micellar particles, amino acids, peptides, polypeptides, proteins, glycoproteins, proteoglycans, ribonucleic acids, glass, silicates, and insoluble ceramics.

In a specific embodiment said said alkaline solution comprises gelatin. In another specific embodiment said alkaline solution comprises hyaluronic acid. In yet another embodiment said alkaline solution comprises silk fibroin. In a further embodiment said alkaline solution comprises at least one biologically active agent. The alkaline solution may for example comprise a drug. In a further embodiment said alkaline solution comprises at least one drug carrier.

In one embodiment of the present invention the construct is heated to the melting point before contacting with the alkaline solution.

In one embodiment the solution is deposited using at least two solutions thereby obtaining a hydrogel comprising compartments corresponding to the different solutions. In one embodiment thereof, said at least two solutions comprise different concentrations of HA and DVS.

In one embodiment of the present invention said alkaline solution comprises NaOH. Said alkaline solution has a pH>10.

In one embodiment said solution is deposited using a device comprising a motor-driven nozzle. In another embodiment said solution is deposited using fused deposition modelling (FDM).

In one embodiment said solution is deposited in at least one layer. For example, said solution is deposited in a layer-by-layer process thereby obtaining a construct comprising one or more layers.

In one embodiment the method according to the present invention further comprises a step of coating the printed construct or the hydrogel with a coating.

In one embodiment the method further comprises a step of washing the hydrogel to obtain a neutral pH of said hydrogel. In one embodiment thereof said hydrogel is washed in water, in a buffer having a pH of about 7 and/or in a solution comprising water and alcohol. Preferably, said hydrogel is washed at least two times. In a particular embodiment the method further comprises a step of washing the hydrogel in water and/or in a buffer having a pH of about 7 and further comprising a step of washing the hydrogel in a solution comprising water and alcohol. The washing step is preferably performed after crosslinking with the alkaline solution.

The present invention also provides a method of preparing a crosslinked HA hydrogel having a predetermined pattern of ordered structural elements, said method comprising the steps of
a. mixing HA, BDDE and a first solvent to obtain a mixture of said HA, BDDE and first solvent;
b. depositing said solution onto a cooled substrate to obtain a frozen construct;
c. lyophilizing the frozen construct to obtain a lyophilized construct;
d. immersing said lyophilized construct in a second solvent
whereby a cross-linked hydrogel is obtained.

Preferably, said HA and BDDE are mixed before addition of the first solvent. In one embodiment said HA is in the form of a powder.

In one embodiment said frozen construct is embedded in a second solution comprising HA before lyophilizing said frozen construct.

The ratio of HA to BDDE is for example in the range of from 10:1 to 2:1 by weight. Preferably, the ratio of HA to BDDE is in the range of from 5:1 to 3:1 by weight.

For example, said first solvent is water. In another embodiment said first solvent is a buffer. Preferably, said first solvent comprises one or more salts. In particular, said first solvent comprises sodium and/or potassium ions. It is preferred that said first solvent comprises salt in a concentration of at least 100 mM.

For example, said second solvent is water. In another embodiment said second solvent is a buffer. Preferably, said second solvent comprises one or more salts. In particular, said second solvent comprises sodium and/or potassium ions. It is preferred that said second solvent comprises salt in a concentration of at least 100 mM.

In one embodiment said second solvent comprises at least one additional component selected from the group consisting of calcium phosphates, synthetic water-soluble polymers, synthetic water insoluble polymers, plant derived carbohydrates, microbial derived carbohydrates, animal carbohydrates, extracellular matrix compounds, chitosan and derivatives thereof, fatty acids, soaps, micellar particles, amino acids, peptides, polypeptides, proteins, glycoproteins, proteoglycans, ribonucleic acids, glass, silicates, and insoluble ceramics. In another embodiment said second solvent comprises gelatine. In yet another embodiment said second solvent comprises hyaluronic acid. In a further embodiment said second solvent comprises silk fibroin. For example, said second solvent comprises at least one drug carrier. In one embodiment said second solvent comprises at least one biologically active agent.

In one embodiment the solution is deposited using at least two solutions thereby obtaining a hydrogel comprising compartments corresponding to the different solutions. For example, said at least two solutions may comprise different concentrations of HA and BDDE.

In one embodiment said solution is deposited using a device comprising a motor-driven nozzle. For example, said solution is deposited using fused deposition modelling (FDM).

In one embodiment said solution is deposited in at least one layer. Said solution may for example be deposited in a layer-by-layer process thereby obtaining a construct comprising one or more layers.

In one embodiment, the method further comprises a step of coating the printed construct or the hydrogel with a coating.

In another aspect, the present invention relates to use of a hydrogel prepared by the method as defined herein, for making a 3D cell culture matrix.

In one embodiment said 3D cell culture matrix is for use in a method of tissue repair and/or cell therapy. Thus, one embodiment relates to a 3D cell culture matrix comprising the hydrogel of the present invention for use in a method of tissue repair and/or cell therapy.

In yet another aspect, the present invention relates to use of a hydrogel prepared by the method as defined herein, for drug development, stem cell research and/or cancer research, or to a hydrogel prepared by the method as defined herein for use in a method of cosmetic surgery or for use as wound dressing in a method of dressing wounds.

A further aspect of the present invention relates to a cross-linked HA hydrogel obtainable by the methods as defined herein.

Preferably, said hydrogel has a loss modulus of at least 10 Pa. In a preferred embodiment said hydrogel has storage modulus greater than the loss modulus.

Yet a further aspect of the present invention relates to a 3D cell culture matrix comprising the hydrogel obtainable by the method as defined herein.

Another aspect of the present invention relates to a wound dressing comprising the 3D cell culture matrix as defined above.

### Description of Drawings

**Figure 1.** Figure 1, upper panel shows the storage modulus at three alkaline solutions comprising different NaOH and NaCl concentrations. Figure 1, lower panel shows the loss modulus at three different NaOH concentrations. For all types of hydrogels it can be seen that G '> G'', and the elastic modulus is therefore greater. Sample visco-elastic properties are dominated by the elastic properties, which mean that the samples have a certain structure and gel strength. It may be inferred from the data that G '(3 M NaCl, 2 M NaOH)> G' (4 M NaCl, 1 M NaOH)> G '(2M NaOH).
**Figure 2****.** SEM micrograph of lyophilized HA with DVS without crosslinking. Elongated microporosity/texture along the the 200 µm wide fibers arise from thermally induced phase separation of water from HA during solution quenching from 35 °C to -25 °C. Gold sputter coated sample. Bar is 1 mm.
**Figure 3****.** SEM micrograph of lyophilized HA-DVS scaffold after crosslinking in alkaline solution comprising 1% HA. The fibers in the hydrogels appear solid and macropores are spanned by strands of crosslinked Hyaluronic acid. In contrast to the hydrogels shown in Figure 2, morphological traces of ice crystal formation have been obliterated. of lyophilized HA-DVS scaffold after crosslinking in alkaline solution comprising 1% HA
**Figure 4****.** Phase contrast micrographs of live cell aggregates in HA-DVS and Algimatrix at 7 days of culture. Left panel: Cell spheroids are visibly dispersed in the pore space between the shadowing printed structures. Right panel: Cell spheroids are more discernible in the homogenous alginate matrix. Bar is 1 mm.
**Figure 5****.** Diagram showing DNA content in HA-DVS scaffolds and Algimatrix at time points, 0, day 1, day 2, day 3 and day 7. The DNA content of HA-DVS scaffolds and Algimatrix dropped rapidly from day 0 to day 1.
**Figure 6****.** Frozen HA-DVS 3D printed mat.
**Figure 7****.** A. Light microscope picture showing scaffold morphology of HA and HAF scaffolds at day 0, 7 and 21. B. Diagram showing the diameter of fibers from HA and HAF scaffolds, respectively, at day 0, 7 and 21. No change was seen in fiber thickness. Bar is 850 µm.
**Figure 8****.** Fluorescent micrographs of mesenchymal stem cells on HAF or HA scaffolds on day 3. Bars are 50 µm. Cells were inspected with confocal microscopy on day 3 to investigate cell spreading on the scaffold fibers and stained with Hoechst nuclear stain and Alexa Fluor 488 Phalloidin to visualize the cytoskeleton and cell outline. Fibronectin coated scaffolds showed more spread out individual cells, while pure Hyaluronic acid scaffolds displayed clustered and rounded cells.
**Figure 9****.** Progression of cell viability on HA scaffolds with and without fibronectin. The cell viability was measured after 3, 7, 14 and 21 days of incubation. Cell viability was measured in triplicates using XTT assay (Thermo Scientific) and normalized to day 3. No proliferation was observed on printed HA scaffolds without fibronectin coating with a statistically significant decrease in viability between day 3 and 21.
**Figure 10****.** Progression of calcium accumulation in cell-free and cell-seeded scaffolds with and without fibronectin. The calcium content was measured at 3, 7, 14 and 21 days of incubation. HA: Hyaluronic Acid scaffold. HA_MSC: Hyaluronic Acid scaffold comprising mesenchymal stem cells. HAF: Hyaluronic Acid scaffold comprising fibronectin. HAF_MSC: Hyaluronic Acid scaffold comprising fibronectin and mesenchymal stem cells.
**Figure 11****.** Printing a hydrogel using a selective laser, wherein a focused light source layer-by-layer scans the rising surface of powder bed of frozen HA, DVS and solvent mixture. The frozen mixture may also comprise frozen alkaline solution.

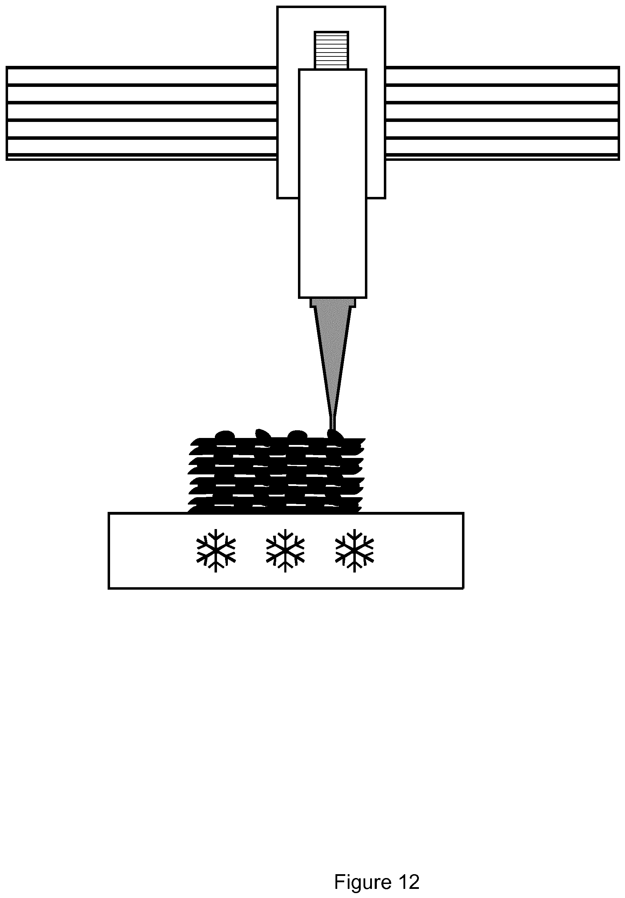

**Figure 12****.** Fused deposition modelling, wherein a nozzle layer-by-layer extrudes liquid HA-DVS solution on a cooled substrate

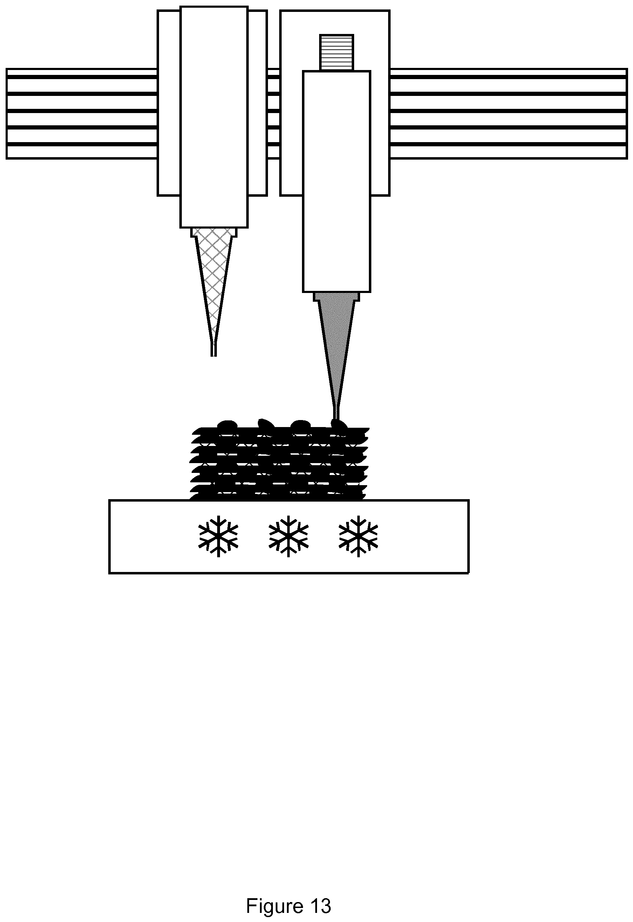

**Figure 13****.** Illustration showing a hydrogel construct, which is made from two materials from at least two nozzles. Preferably, the first material is a HA-DVS solution and the second material is an alkaline solution.

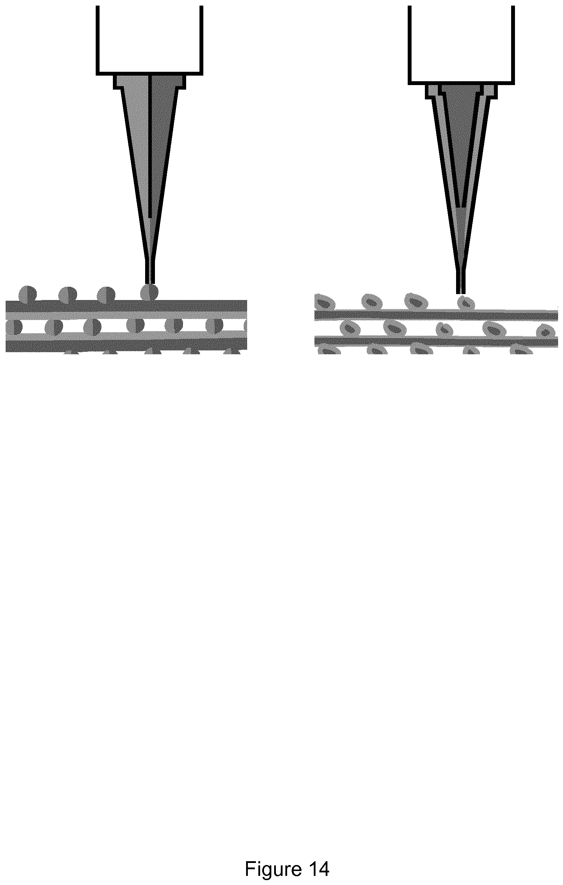

**Figure 14****.** Illustration showing the cross section of the hydrogel wherein the individual fibers are divided into separate material compartments.
**Figure 15****.** A: Illustration showing one layer comprising alternating straight and coiled fibres. B: Illustration showing multiple layers comprising coiled and straight fibres.
**Figure 16****.** A: Illustration showing one layer of particles in parallel rows. B: Illustration showing multiple layers of particles in parallel rows.
**Figure 17****.** A: Macro photograph of lyophilized, fused particle HA-DVS construct showing particles and pores. The construct is 2.8 mm wide. Edge shadows are artefacts. B: Oblique view macro photograph of lyophilized, fused particle HA-DVS construct showing particles and pores and layers. The construct is 2.8 mm wide.
**Figure 18****:** A: Scanning electron micrograph of lyophilized, gold coated, HA-DVS construct comprising fused particles(/droplets/globules). The construct is viewed from the top face. The bar is 1 mm. B: Scanning electron micrograph of lyophilized, gold coated, HA-DVS construct comprising fused particles(/droplets/globules). In this embodiment the construct is 10 particles wide and long, and four layers high. The micrograph is taken at an oblique angle to the construct. The bar is 1 mm.
**Figure 19****:** A: Photograph of HA-BDDE constructs. B: SEM micrograph of lyophilized, gold coated, HA-BDDE construct. The structure and pattern of fiber, lines, and layers is identical to those for the HA-DVS constructs. Bar is 1 mm.

### Detailed description

It is an object of the present invention to provide a hyaluronic acid (HA) hydrogel, such as a three-dimensional (3D) hydrogel, having a well-defined, ordered and homogeneous structure and methods for producing a such HA hydrogels. The hydrogels produced by the methods of the present invention are flexible, have a good tensile strength and have a more homogeneous structure than HA hydrogels generated by known methods. The hyaluronic acid hydrogels of the present invention have a well-defined geometry and an ordered structure allowing the production of more complex 3D hydrogels. The hydrogels produced by the methods of the present invention comprise FDA approved crosslinking agents without trace of additional viscosity enhancers.

Accordingly, one aspect of the present invention relates to a hydrogel comprising a coherent, porous structure of ordered structural elements, wherein said structural elements comprises crosslinked hyaluronic acid.

The terms "hydrogel", "three-dimensional hydrogel" and "scaffold" as used herein refer to the crosslinked construct. Preferably, the hydrogel is a water swollen polymer network.

The term "ordered structural elements" as used herein refers to structural elements that form a specific pattern or network. The specific pattern or network of the hydrogels can be controlled by the methods as described herein, which is used to produce the hydrogels of the present invention. Thus, the methods of the present invention allows for the productions of hydrogels with a predetermined pattern of ordered structural elements.

The hydrogel comprises at least one layer. Each layer comprises or consists of ordered structural elements. Said hydrogel comprises multiple layers.

In one embodiment the ordered structural elements form a hierarchical structure. In a particular embodiment the hydrogel comprises or consists of a hierarchical scaffold or, more preferably, a three-dimensional (3D) hierarchical scaffold.

It is preferred that the pores of the network are interconnected. Thus, in one embodiment said porous structure comprises interconnected pores. In one embodiment at least 50 % of the pores are interconnected. In a more preferred embodiment at least 60%, such as at least 70%, at least 80%, such as at least 90% or at least 95% of the pores are interconnected.

In one embodiment said pores have a width in the range of from 0.05 millimetre (mm) to 5 mm. More preferably said pores have a width in the range of from 0.1 mm to 3 mm, such as for example from 0.5 mm to 2 mm or such as for example from 1 to 2 mm.

In another embodiment the pores have a width in the range of from 0.05 millimetre (mm) to 2 mm, such as for example from 0.05 mm to 1 mm or such as from 0.05 mm to 0,5 mm.

In yet another embodiment the pores have a width in the range of from 0.5 mm to 5 mm such as from 0.5 mm to 3 mm or such as for example from 0.5 mm to1 mm.

The structural elements of the hydrogel can for example be fibres or filaments. These fibres or filaments may comprise substructures such as pores, longitudinal and/or circumferential grooves. The structural elements can also be particles. The particles preferably have the form of small spheres or drops. Thus, in one embodiment the hydrogel comprises or consists of fibres. In another embodiment the hydrogel comprises or consists of particles. In yet another embodiment the hydrogel comprises or consists of fibres and particles.

The fibres have a diameter or thickness in the range of from 50 µm to 500 µm, such as for example from 50 µm to 400 µm or more preferably in the range of from 100 µm to 300 µm.

For example the fibres have a diameter or thickness of about 500 µm, about 400 µm, about 300 µm, about 100 µm or for example about 50 µm.

In a preferred embodiment said fibres have a diameter of about 200 µm.

As described above, the method of the present invention allows the generation of hydrogels comprising fibres that form an ordered structure or pattern. Thus, the direction of the fibres can be controlled by the method of the present invention. It is for example possible to obtain hydrogels comprising fibres that are parallel.

Thus, in one embodiment the hydrogel of the present invention comprises a plurality of parallel fibres. Preferably, the plurality of parallel fibres is within the same layer. For example, at least 60 %, such as at least 70 %, or more preferably at least 80 % or even more preferably at least 90 % or at least 95 % of the fibres within one layer are parallel. In another embodiment the hydrogel comprises at least one layer, wherein said layer consists of parallel fibres.

The hydrogels can also comprise one or more layers wherein the fibres are not parallel.

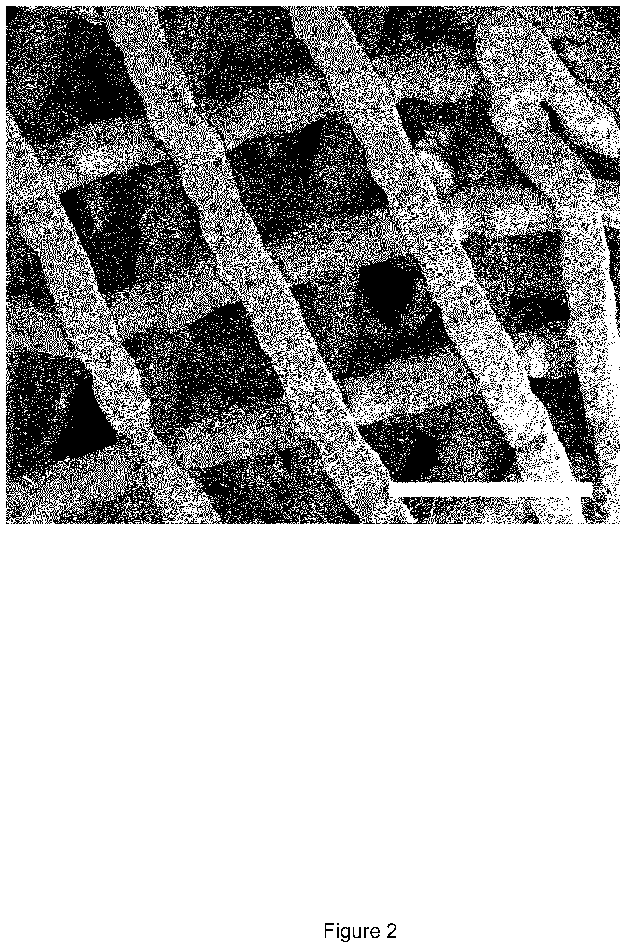

The hydrogel comprises multiple layers. One embodiment thereof, relates to a hydrogel comprising parallel fibres within the same layer, but wherein fibres between neighbouring layers are not parallel. According to the invention fibres between neighbouring layers are not parallel. An example of this embodiment is shown in Figure 2.

For example, fibres, in particular straight fibres, between neighbouring layers may form an angle of at least 10°, such as at least 20°, at least 40°, at least 60°, such as for example at least 80°.

On one embodiment the fibres, in particular straight fibres, between neighbouring layers form an angle between 10° and 90°, such as for example between 20° and 90°, such as between 40° and 90°, such as between 60° and 90°. In one embodiment the fibres between neighbouring layers form an angle of about 60°, about 70°, about 80° or about 90°.

In one embodiment the hydrogel comprises a plurality of non-intersecting fibres within the same layer. For example, at least 60 %, such as at least 70 %, or more preferably at least 80 % or even more preferably at least 90 % or at least 95 % of the fibres within one layer are non-intersecting. In another embodiment the hydrogel comprises at least one layer, wherein said layer consists of non-intersecting fibres.

The fibres are for example straight fibres. However, the fibres can also be coiled fibres. The fibres may for example coiled fibres, wherein each fibre forms a periodic curve. In one embodiment each fibre forms a sinusoidal curve.

In one embodiment the hydrogel comprises straight fibres and coiled fibres. For example, the hydrogel gel comprises multiple layers wherein at least one layer comprises or consists of straight fibres and at least one layer comprises or consists of coiled fibres. For example, the hydrogel may comprise alternating layers of straight fibres and coiled fibres.

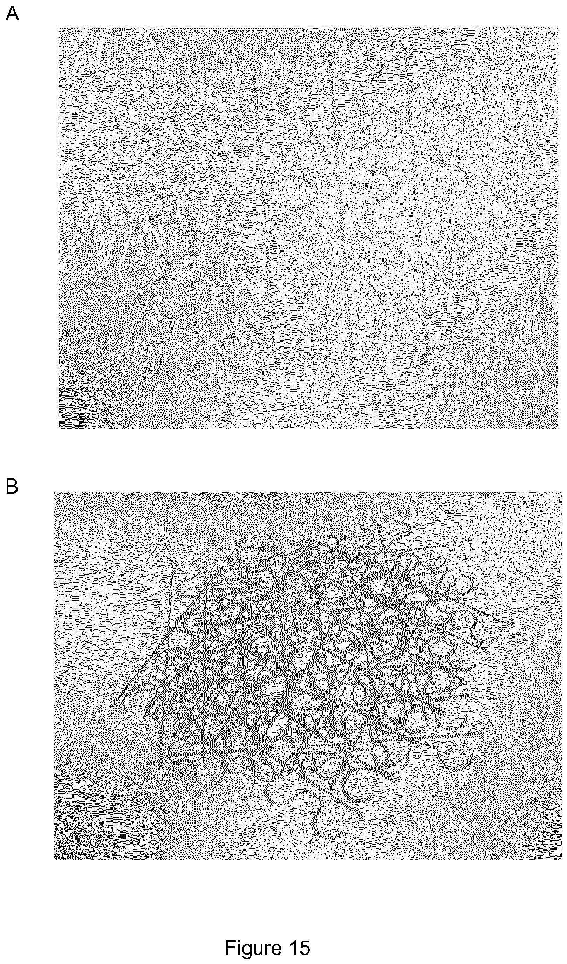

The fibres within at least one layer consist of alternating coiled and straight fibres. Alternating coiled and straight fibres for example includes patters of (1 coiled fibre + 1 straight fibre) x n, wherein n is an integer of at least one or at least 2, at least 3 such as at least 4 or at least 5. An example of this embodiment is illustrated in Figure 15A. However, alternating coiled and straight fibres can also include patters of (1 coiled fibre + 2 straight fibres) x n or for example patters of (2 coiled fibres + 1 straight fibre) x n or for example patters of (x coiled fibres + y straight fibres) x n, wherein x and y are integers of at least 1.

The particles can for example be in the form of a sphere, a droplet or an ellipsoid.

The particles have a diameter or a maximum thickness in the range of from 10 micrometre (µm) to 1 mm, such as from 30 µm to 1 mm, such as for example from 50 µm to 800 µm, such as from 100 µm to 600 µm or such as from 200 µm to 400 µm.

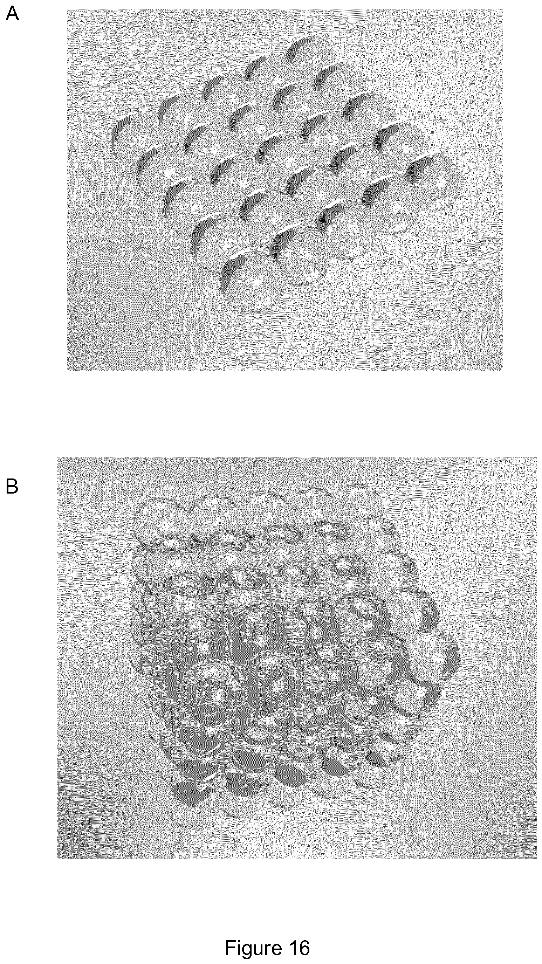

The particles are aligned in rows. In one embodiment thereof the hydrogel comprises multiple layers, wherein each layer comprises multiple rows of particles. Preferably said rows are parallel. The particles may also be arranged in rows that are continuous through at least two layers. Examples of these embodiments are shown in Figure 16.

The hydrogel or scaffold can for example have the shape of a mat, a sheet, a cube, a cuboid, a sphere, an ellipsoid, a prism or a cylinder. In one embodiment the hydrogel is solid. In another embodiment the hydrogel is hollow. That is, in one embodiment the hydrogel forms a shell. The shell can for example have the above mentioned shapes. The shape of the hydrogel is the macroscopic shape or architecture of the hydrogel. It is the structural elements that form the shape. The structural elements and the ordered pattern formed by the structural elements are as defined elsewhere. Thus, the pattern formed by the individual structural elements is within and between the layers of the hydrogel. The macroscopic shape or architecture of the hydrogel is formed be the layers that comprise structural elements in a well-defined order as described herein.

The shell of the hydrogel preferably have a thickness in the range of from 0.05 mm to 7 mm, such as for example in the range of from 0.1 mm to 5 mm, such as from 0.2 mm to 2 mm or such as for example from 0.5 mm to 1 mm.

The hydrogels may in addition to the ordered structural elements also comprise one or more layers that are obtained by for example spraying, electospraying, melt spinning, electrospinning or casting. This will results in a layer not comprising ordered elements.

The hyaluronic acid is crosslinked with DVS, BDDE or tyramine. Thus, in one embodiment the hydrogel comprise structural elements that comprise or consists of hyaluronic acid crosslinked with DVS. In another embodiment the hydrogel comprise structural elements that comprise or consists of hyaluronic acid crosslinked with BDDE. In yet another embodiment the hydrogel comprise structural elements that comprise or consists of hyaluronic acid crosslinked with tyramine. In yet another embodiment the hydrogel comprise structural elements that comprise or consist of hyaluronic acid crosslinked with BDDE and structural elements that comprise or consists of hyaluronic acid crosslinked with DVS. Structural elements that comprise or consist of hyaluronic acid crosslinked with BDDE or DVA can also be combined with structural elements that comprise or consist of hyaluronic acid crosslinked with tyramine.

The hydrogels of the present invention can be applied for medical use and/or therapy. It is therefore preferred that the hydrogels are biocompatible.

For example, the hydrogel can have a shape of an anatomical structure. This shape can be generated from medical 3D imaging data for example CT, MRI or ultrasound.

The hydrogels according to the invention may comprise at least one additional component. In a preferred embodiment said component is a biologically active agent. The biologically active agent can for example be a drug. The biologically active agent can be loaded into the hydrogel and then released over a specified period. Thus, the hydrogels provide a possibility for sustained release of the biologically active ingredient or the drug. Preferably, the biologically active agent dispersed or dissolved uniformly throughout the structure of the hydrogel

The additional component is preferably homogeneously dispersed in the pores of the hydrogel. Preferably the pores are interconnected.

In one embodiment the at least one additional component is selected from the group consisting of calcium phosphates, synthetic water-soluble polymers, synthetic water insoluble polymers, plant derived carbohydrates, microbially derived carbohydrates, animal carbohydrates, extracellular matrix compounds, chitosan and derivatives thereof, fatty acids, soaps, micellar particles, amino acids, peptides, polypeptides, proteins, glycoproteins, proteoglycans, ribonucleic acids, glass, silicates, and insoluble ceramics.

The hydrogels or scaffolds of the present invention can be created to resemble those found in nature. The methods of the present invention allows for the production of 3D hydrogels that possess proper shape, dimensions, porosity and physical properties suitable for cell attachment and subsequent tissue development.

Thus, in one embodiment of the present invention the hydrogel further comprises cells. It is preferred that the cells are mammalian cells such as human cells. When cells attach the scaffold cellular growth and subsequent tissue development can occur. This can be done in a cell medium comprising nutritional requirements necessary for cellular growth.

It is preferred that these additional components are covalently linked to the hydrogel. In another embodiment said additional components are linked to the scaffold by coacervation, electrostatic interactions, hydrogen bonding and/or van der Waals forces. In one embodiment the hydrogel comprises a coating. Examples of coatings are described elsewhere herein. The coating can for example be fibronectin.

### Method for producing an HA hydrogel crosslinked with DVS

The hydrogels as described herein above can for example be crosslinked with DVS. A method for producing a hydrogel according to the present invention and wherein HA is crosslinked with DVS is described below.

Thus, in one aspect, the present invention relates to a method of preparing a crosslinked HA hydrogel having a predetermined pattern of ordered structural elements as described herein comprising the steps of
a. mixing HA, divinyl sulfone (DVS) and solvent to obtain a mixture of said DVS, HA and solvent wherein said solvent has a pH below 9;
b. depositing said solution onto a cooled substrate to obtain a frozen construct;
c. contacting (or immersing) said frozen construct, which has not yet been crosslinked, with (in) an alkaline solution having a pH above 10 at a temperature below the freezing point of the construct to cross-link HA with DVS,
whereby a crosslinked HA hydrogel is obtained.

In one embodiment thereof, the present invention relates to a method of preparing a hydrogel comprising the steps of
d. mixing HA powder with divinyl sulfone (DVS) to obtain a mixture of said DVA and HA;
e. adding a solvent to said mixture to obtain a solution comprising HA and DVS;
f. depositing said solution onto a substrate to obtain a construct;
g. contacting said construct with an alkaline solution to cross-link HA with said DVS,
whereby a hydrogel is obtained.

Preferably, said hydrogel and said construct is three-dimensional.

Thus, in this embodiment HA powder is mixed with DVS to obtain a loose paste. In one embodiment the HA powder is mixed with DVS before adding the solvent.

The term "construct" or "three-dimensional construct" as used herein refers to the printed or deposited HA-DVS solution, which has not yet been crosslinked.

The term "HA-DVS solution" as used herein means a solution comprising HA, DVS and solvent. The HA-DVS solution may also be referred to as a HA-DVS composition or a mixture of HA, DVS and solvent

Preferably the HA, DVS and solvent is mixed with stirring for at least 1 minutes, at least 10 minutes, at least 30 minutes or for example at least 1 hour.

The ratio of HA to DVS may for example be in the range of from 20:1 to 1:1 by weight, such as for example from 20:1 to 2:1 by weight, or in the range of from 15:1 to 2:1 by weight, such as for example from 12:1 to 2:1 by weight, for example from 10:1 to 2:1 by weight, or in the range of from 8:1 to 2:1 by weight, such as for example from 6:1 to 2:1 by weight, for example from 5:1 to 2:1 by weight, from 4:1 to 2:1 or such as for example from 3:1 to 2:1 by weight.

In one embodiment ratio of HA to DVS is in the range of from 10:1 to 2:1 by weight.

In another embodiment the ratio of HA to DVS may for example be in the range of from 20:1 to 3:1 by weight, such as for example from 15:1 to 3:1 by weight, such as for example from 12:1 to 3:1 by weight, for example from 10:1 to 3:1 by weight, or in the range of from 8:1 to 3:1 by weight, such as for example from 6:1 to 3:1 by weight, for example from 5:1 to 3:1 by weight, or such as for example from 4:1 to 3:1 by weight.

In a preferred embodiment the ratio of HA to DVS is in the range of from 5:1 to 3:1.

The ratio of HA to DVS may for example be 15:1, 10:1, 8:1, 6:1, 5:1, 4:1, 3:1 or 2:1 by weight. In a preferred embodiment the ratio of HA to DVS is 5:1, 4:1 or 3:1 by weight.

The HA-DVS solution may for example comprise at least 15 percentage by weight (wt%) HA and 3 wt% DVS, such as at least 10 wt% HA and 3 wt% DVS or such as for example at least 5 wt% HA and 3 wt% DVS.

In another embodiment the HA-DVS solution comprises at least 15 wt% HA and 2 wt% DVS, such as at least 10 wt% HA and 2 wt% DVS or such as for example at least 5 wt% HA and 2 wt% DVS.

In yet embodiment the HA-DVS solution comprises at least 15 wt% HA and 1 wt% DVS, such as at least 10 wt% HA and 1 wt% DVS or such as for example at least 5 wt% HA and 1 wt% DVS.

In a preferred embodiment the HA-DVS solution comprises 5 wt% HA and 1 wt% DVS.

In one embodiment, the HA or salt thereof has a molecular weight in the range of between 100000 Dalton (Da) and 3000000 Da; for example in the range of between 300000 Dalton (Da) and 3000000 Da; preferably in the range of between 400000 Da and 2500000 Da; more preferably in the range of between 500000 Da and 2000000 Da; and most preferably in the range of between 600000 Da and 1800000 Da. In a highly preferred embodiment, the molecular weight of HA is 700000 Da.

In one embodiment, the HA or salt thereof is derived from animal sources for example rooster comb. In a preferred embodiment, the HA or salt thereof is produced by bacterial fermentation.

HA and DVS and solvent is mixed to obtain a mixture of said DVS, HA and solvent or an HA-DVS solution.. In one preferred embodiment said solvent comprises a buffer, such as a pH-buffer. In a preferred embodiment said buffer is acidic. Said buffer has a pH below 9. It is preferred that said buffer has a pH below 8. In another preferred embodiment said buffer has a pH below 7. In another embodiment the pH of the solvent or buffer is below 6, such as for example below 5 or below 4. In a preferred embodiment said solvent is a buffer, preferably a buffer as described above.

In one embodiment said solvent or buffer comprises an antioxidant.

Preferably, said buffer or solvent comprises one or more salts.

In one embodiment said buffer or solvent comprises salts with monovalent cations, such as Li⁺, Na⁺, K⁺. In another embodiment the buffer or solvent comprises salts with divalent cations such as for example Be²⁺, Mg²⁺, Ca²⁺, Sr²⁺. In a preferred embodiment, the anion is inorganic such as Cl⁻. In one embodiment said solvent comprises NaCl, KCI and/or LiCI

The concentration of the salt may range from 10 millimole/Litre (mM) to 3 M, preferably from 0.5 M to 2 M or more preferably from 1 to 4 M or from 1 M to 3 M.

In one embodiment the concentration of salt is at least 10 mM, such as at least 50 mM, at least 100 mM, at least 500 mM or more preferably at least 1 M, at least 2 M or at least 3 M.

The concentration of salt may for example be 500 mM, 1 M, 2 M, 3 M or 4 M.

In another embodiment, the solvent comprises or consists of a buffer, which comprises one or more members of the sodium phosphates where the anion may be organic such as PO₄³⁻.

In another embodiment, the buffer may comprise a potassium phosphate buffer. In one out of many embodiments, the solvent may comprise an acetate buffer, a citrate buffer, or an amine buffer, for example Tris or BIS-TRIS, zwitterionic buffers, for example HEPES or DIPSO.

In one embodiment the solvent comprises or consists of biological buffers selected from the group consisting of MES, BIS-TRIS, ADA, PIPES, ACES, MOPSO, BIS-TRIS Propane, BES, MOPS, TES, HEPES, DIPSO, TAPSO, Trizma, POPSO, HEPPS , TRICINE, GLY-GLY. Biological buffers can be useful for modulating the crosslinking reaction.

In one embodiment said buffer or solvent comprises lithium, sodium, potassium, phosphate, chloride and/or bromide ions. In particular, said buffer or solvent comprises sodium and/or potassium ions. In one embodiment said buffer or solvent comprises sodium and/or potassium ions in a concentration of at least 100 mM at least 500 mM or more preferably at least 1 M, at least 2 M or at least 3 M.

At low pH aqueous HA solutions increase viscosity due to protonation of the carboxylate groups. The increased viscosity is advantageous for 3D printing because it increases the potential feature resolution of the scaffold (see below). Further, the low pH inhibits HA crosslinking which in turn lowers probability of crosslinking in the dispenser and nozzle. Degradation of HA is accelerated at low pH, but this can be mitigated by lowering the solution temperature.

A viscous solution comprising HA and solvent can for example be prepared prior to adding DVS in said preferred ratios. The resulting viscosity will require more thorough and forceful agitation to achieve even mixing of the DVS in the solution.

Before depositing the HA-DVA solution onto the substrate, said solution is preferably kept at or brought to a temperature above its melting point in order to maintain a fluid state of the solution. In one embodiment the HA-DVS solution is kept at or heated to a temperature above -25 °C, such as for example -10 °C. In a more preferred embodiment the said HA-DVS is kept at or heated to a temperature above 0 °C, such as for example above 10 °C, such as for example above 20 °C, such as for example above 30 °C.

The HA-DVS solution is deposited onto a substrate to obtain a construct, preferably a three-dimensional (3D) construct. Preferably the construct changes into the solid from when it is deposited onto the substrate. Thus, the temperature of the substrate will solidify the HA-DVS solution. Thus, it is preferred that the HA-DVS solution is cooled to a temperature below its melting point during and/or after it has been deposited onto the substrate to solidify the solution and create a construct that is dimensionally stabilized. When the hydrogel comprises multiple layers, it is preferably only the first layer that is deposited directly onto the substrate. The second layer will preferably be deposited onto the first layer, thereby obtaining the temperature of the first layer which will solidify the second layer. A third layer can then be deposited onto the second layer and so on. It is important that the HA-DVS solution or the layers are cooled to obtain a temperature below the gel point of the solution such that the layers are solidified.

The HA-DVS can for example be cooled to a temperature below 10 °C, such as for example below 5 °C. In a more preferred embodiment the HA-DVS solution is cooled to a temperature below 0°C, such as for example below -5°C, such as below -10°C, such as for example below -15°C. In a preferred embodiment the HA-DVS solution is cooled to a temperature below -20°C. The HA-DVS may also be cooled to a temperature below -25°C, such as for example below -30°C, below -40°C or for example below -50°C.

The substrate can for example be selected from a solid plate, porous plate, mesh, thin film, particle bed and a membrane, such as an elastic, flexible, bendable or stretchable membrane. The composition of these may comprise: HA, graphite, pyrolytic carbon, silicon, water ice, water ice particles a metal surface, a metal oxide surface, a metal nitride surface, an inert high density liquid such as mercury or eutectic alloys, polymer, elastomers, ceramics, glass, or composites thereof.

The substrate may be liquid cooled, air cooled, or thermoelectrically cooled or combinations thereof. In one embodiment the substrate is cooled using dry ice.

Addition of salt and/or buffer to the HA-DVS solution will increase osmolarity and decrease the temperature at which the solution solidify. DVS has a melting point at -26 °C and above this temperature it will likely be trapped as liquid microdroplets dispersed among icecrystals.

It is preferred that the construct or hydrogel is kept at a temperature below its melting point at least until chemical cross-linking has taken place to maintain the structure of the construct.

However, before the HA-DVS solution is deposited onto the substrate it should preferably be at a temperature above the gel point such that the solution is liquid. In one embodiment the HA-DVS solution is heated to a temperature above -25 °C, such as for example -10 °C. In a more preferred embodiment the said HA-DVS is heated to a temperature above 0 °C, such as for example above 10 °C, such as for example above 20 °C, such as for example above 30 °C.

The solution comprising solvent, HA and DVS is deposited onto a substrate to obtain a construct, preferably a three-dimensional (3D) construct. Thus, preferably the construct is in a solid state. The solvent can for example be deposited by printing or plotting. This can for example be done by using a deposition device comprising a laser or a nozzle. The nozzle may also be referred to as a syringe or a needle. Preferably, the solvent is deposited by 3D printing. Thus, in one embodiment the solvent is deposited onto the substrate by using a 3D printer. The construct can be produced by extruding small strings or filaments of the solution to form layers as the solution solidify immediately after extrusion from for example a nozzle. In a preferred embodiment said solution is printed using a device comprising a motor-driven nozzle.

In another embodiment the HA-DVS solution is prepared as a powder and distributed in a substrate as described. This can be done by selective laser sintering (SLS).

Thus, in one embodiment the HA-DVS solution is deposited onto the substrate by using selective laser sintering (SLS). This will result in the formation of a construct, preferably a 3D construct. In this embodiment HA, DVS and solvent are prepared as a fine solid, powdery mixture and distributed evenly on a refrigerated substrate. A laser or other focused heat source will then scan a pattern on the powder to fuse the mixture in successive layers. At the end of the printing the solid printed construct is removed from the unfused powder and blown with cold dry gas. For low viscosity HA-DVS solutions, the solid printed construct can be printed using a multi-nozzle.

In a preferred embodiment, the HA-DVS solution can be deposited, extruded or printed onto the substrate by fused deposition modelling (FDM). Fused deposition modelling (FDM) is an additive manufacturing (AM) technology commonly used for modelling, prototyping, and production applications. The technique is commonly used for 3D printing. In this process the HA-DVS solution is dispensed on a cooled substrate onto which it will solidify or fuse with underlying material. The substrate is preferably cooled to a temperature below the melting-point of the HA-DVS solution. At low pH aqueous HA solutions increase viscosity due to protonation of the carboxylate groups. The increased viscosity is desirable for printability by FDM because it increases the potential feature resolution of the scaffold. Further, the low pH inhibits HA crosslinking which in turn lowers probability of gelation in the nozzle. Degradation of HA is accelerated at low pH, but this can be mitigated by lowering the solution temperature.

The nozzle can for example be moved with at least two degrees of freedom or at least three of freedom. In one embodiment the nozzle can be moved in both horizontal and vertical directions by a numerically controlled mechanism. The nozzle follows a tool-path controlled by a computer-aided manufacturing (CAM) software package, and the part is built from the bottom up, one layer at a time. Thus, the dimensions and shape of the scaffolds or constructs are defined by the CAD drawing, which controls the path of the 3D printer. In addition, individual constructs can be cut from larger scaffold constructs by for example punching, cutting and/or cleaving.

In one embodiment said solution is deposited or printed in a layer-by-layer process thereby obtaining a construct comprising one or more layers. Thus, after depositing or printing the first layer of HA-DVS solution, additional layers can be deposited or printed on the first layer.

In one embodiment the construct is deposited using at least two solutions thereby obtaining a hydrogel comprising compartments corresponding to the different solutions.

This is done by using two nozzles. Thereby, three-dimensional constructs comprising compartments corresponding to the different solutions are obtained.

Preferably this is done by printing, such as FDM. In one embodiment said at least two solutions comprise different concentrations of HA and/or DVS. Thus, in one embodiment the construct is deposited using two solutions, solution 1 and solution 2. Solution 1 may for example comprise a higher concentration of HA than solution 2. In another embodiment solution 1 comprises a higher concentration of DVS than solution 2. In yet another embodiment solution 1 comprises higher concentrations of DVS and HA than solution 2. Preferred concentrations of DVS and HA are described herein above. In one embodiment solution 2 does not comprise HA and/or DVS. For example, in one embodiment, solution 1 may comprise HA and DVS and solution 2 may comprise an alkaline solution such as HA and NaOH. In another embodiment the molecular weight of HA in solution 1 is different from the molecular weight of HA in solution 2.

For example, in FDM to create construct shapes or internal porosities with large overhangs or arches, a sacrificial support structure is advantageous. Having at least one nozzle depositing a sacrificial support material is therefore preferable. This, in a preferred embodiment the innermost material may comprise a material with a melting point below 10 °C and a high vapor pressure as a solid. For example, the innermost material can be water, eutectic mixtures of camphor and menthol. For example a vapor pressure higher than 10 Pa.

In one embodiment, the support material comprises a HA solution without crosslinking agent. The HA solution is preferably viscous and dilute. The support material can be removed by washing following crosslinking of the supported hydrogel.
The solvent-rich support material can disintegrate during or following lyophilisation of the frozen construct prior to crosslinking. This is highly advantageous as no additional raw materials are needed. The pH of the HA solution can for example be lowered with carbonic acid.

In another preferred embodiment, the innermost material in the said concentric codeposition is a carrier for a biomaterial, which will be encapsulated by the outer layer of HA hydrogel.

In one embodiment the solution is printed in inert gas atmosphere. Preferably the inert gas atmosphere is temperature-regulated. For example, the ambient temperature can be regulated.

Further methods for printing 3D hydrogels are described in EP2679669.

Additional components can be added to the HA-DVS solution to provide further properties to the construct. In one embodiment the HA-DVS solution comprises at least one additional component. Preferable said additional component is a biomaterial. The biomaterial is in one embodiment selected from the group consisting of calcium phosphates, synthetic polymers, plant derived carbohydrates, microbially derived carbohydrates, animal carbohydrates, extracellular matrix compounds, chitosan and derivatives thereof, fatty acids, soaps, micellar particles, liposomes, amino acids, peptides, polypeptides, proteins, glycoproteins, proteoglycans, ribonucleic acids, bioglass, silicates, and insoluble ceramics.

The alkaline solution has a pH above 10. It is preferred that the alkaline solution comprises salt, such as KCI, LiBr and/or LiCI. The presence of salt in the alkaline solution will decrease the freezing point of the alkaline solution which is important to keep the alkaline solution in the liquid form during crosslinking of the printed frozen constructs.

In one embodiment the alkaline solution comprises salt at a concentration of at least 100 mM. In a preferred embodiment the alkaline solution comprises salt at a concentration of at least 200 mM, such as at least 300 mM, such as for example at least 500 mM, such as at least 800 mM, at least 1 M, such as for example at least 2 M, such as at least 3 M, at least 4 M or such as for example at least 5 M.

In one embodiment the alkaline solution comprises base at a concentration of at least 100 mM. In a preferred embodiment the alkaline solution comprises base at a concentration of at least 200 mM, such as at least 300 mM, such as for example at least 500 mM, such as at least 800 mM, at least 1M, such as for example at least 2 M, such as at least 3 M, at least 4 M or such as for example at least 5 M.

In one embodiment the alkaline solution comprises salt and base at a total concentration of at least 100 mM. In a preferred embodiment the alkaline solution comprises salt and base at a total concentration of at least 200 mM, such as at least 300 mM, such as for example at least 500 mM, such as at least 800 mM, at least 1M, such as for example at least 2 M, such as at least 3 M, such as for example at least 4 M or such as for example at least 5 M..

The base can for example be selected from the group consisting of NaOH, LiOH, KOH, Mg(OH)₂, Ca(OH)₂ Sr(OH)₂, Ba(OH)₂.

Examples of hydrogels comprising different concentration of base and salt are presented in the example section.

In one embodiment said alkaline solution comprises gelatin. In another embodiment said printing solution and/or said alkaline solution comprises hemagglutinin. In yet another embodiment said printing solution and/or said alkaline solution comprises silk fibroin

In a preferred embodiment said additional component is in particle form. Thus, the additional component may be a particle. The particles can for example be selected from nanoparticles, microparticles and macroparticles.

Crosslinked, highly swollen HA- hydrogels are transparent and colourless. In some applications it is useful to increase the visibility of the hydrogel to the naked eye such as for example in phase contrast microscopy and fluorescence imaging.

The alkaline solution may comprise at least one additional component. In one embodiment said component is a biologically active agent.

In one embodiment said alkaline solution comprises at least one additional component selected from the group consisting of dyes, fluorescent dyes, fluorophores, pigments, radiocontrast agents, quantum dots, heavy metal free quantum dots, carbon nanoparticles, ceramic particles, glass particles, fused silica particles, metal particles, metal oxide particles, metal nitride particles. Again, the particles may be selected from nanoparticles, microparticles and macroparticles.

The visibility of the scaffold or hydrogel can also be increased by altering the transparency of the hydrogel without adding fluorophores or pigments. Thus in one embodiment, the optical opacity of the hydrogel is increased by increasing the concentration of HA and/or DVS and/or osmolarity of the HA-DVS solution.
In another embodiment, the HA-DVS hydrogel is embedded in or framed by an opaque HA-DVS hydrogel. This can improve visual location of the hydrogel in for example a cell culture container.

In one embodiment said alkaline solution comprises at least one drug carrier. Said drug carrier may for example be selected from the group consisting of carrier proteins, liposomes, polymeric micelles, microspheres, carbon nanoparticles, nanosponges, nanofibers, protein-DNA complexes, protein-drug conjugates, hemagglutinin-drug conjugates, virosomes, dendrimers, cyclodextrins, mesoporous silica, phyllosilicates.

It is preferred that these additional components will be covalently linked to the hydrogel.

In one embodiment said additional components are linked to the scaffold by coacervation, electrostatic interactions, hydrogen bonding, or van der Waals forces.

The dimensions of the crosslinked, hydrated scaffold will reproducibly vary from those of the solid frozen printed construct, which must be compensated for in the CAD drawing. In preferred embodiments, the dimensions of the construct matches well sizes of specific multiwell plates, to enable standardized, high throughput, 3D cell culture/screening. For cylindrical scaffolds, the diameter can range from 0,5 mm to 100 mm, preferably from 5 mm to 40 mm.

When the printing process or deposition step is finished the construct must be kept below the melting point to stabilize the structure of the construct. When un-cross-linked, it will melt at room temperature. Before cross-linking, the printed construct or scaffold can be preserved by lyophilisation. Lyophilisation is a dehydration process that includes freeze-drying the construct by freezing the material and then reducing the surrounding pressure to allow the frozen water in the construct to sublimate directly from the solid phase to the gas phase. Thus, in one embodiment the construct is lyophilized prior to cross-linking. In this embodiment, as the construct is dry, crosslinking can occur at any e.g. room temperature. The presence of salt modifies the mechanical characteristics of the finished hydrogel.

Cross-linking is initiated by contacting said construct with an alkaline solution. In a preferred embodiment the alkaline solution comprises NaOH. In another preferred embodiment the alkaline solution comprises LiOH, KOH, Mg(OH)₂, Ca(OH)₂ Sr(OH)₂, Ba(OH)₂.

Ion one embodiment the hydrogel is saturated or partially saturated with cations such as for example Ca²⁺ and/or Sr²⁺.

Said alkaline solution has a pH>10. In a preferred embodiment the alkaline solution has a pH>14. In a more preferred embodiment, alkaline solution the concentration of hydroxyl ions is higher than 1 M.

In one embodiment the construct is cross-linked by contacting said construct with a cooled alkaline solution. Preferably, the alkaline solution is cooled to a temperature below the melting-point of the frozen construct. Contacting said construct with a cooled alkaline solution is preferably done when the construct has not been lyophilized. Because of the pore interconnectivity, the liquid alkaline solution will distribute evenly and rapidly inside the construct. At low temperatures, the diffusion of hydroxyl ions into the frozen construct is slow and the cross-linking process is therefore slow. Raising the temperature of the alkaline solution will lead to a homogeneous cross-linking of the porous construct.

The construct can be contacted with the alkaline solution by for example dipping or immersing the construct in an alkaline solution bath.

Directly cross-linking the construct, without performing a lyophilization step, can be advantageous because it is simple. Further, during the freezing of the HA-DVS solution thermally induce phase separation (TIPS) will take place. TIPS will rearrange the previously homogeneous HA solution into oriented HA rich and water rich domains which will form ice crystals. This, specific microstructure will be fixed at cross-linking and the obtained microporosity can be advantageous for some applications. For optimal mechanical properties it is preferred that the water and/or HA domains are as interspersed as possible or spread homogeneously throughout the hydrogel or scaffold.

The method of the present invention may also include an annealing step wherein the construct is carefully heated to the melting point and then re-solidified in cold alkaline solution. In one embodiment the construct is heated to the melting point before contacting with the alkaline solution.

After cross-linking of the construct, the cross-linked hydrogel is preferably neutralized by contacting said hydrogel with an acidic solution. This can for example be done by acidic titration such as for example HCI titration. The hydrogel may in one embodiment be imbedded or immersed in an acidic bath. In another embodiment the hydrogel is washed in water and/or a neutral buffer, preferably distilled water. The hydrogel can for example be washed in Phosphate-Buffered Saline (PBS).

The hydrogel can for example be kept in PBS at a neutral PH.

In one embodiment the method of the present invention further comprises a step of coating the printed construct or the hydrogel with a coating such as a biomaterial coating. The coating can be applied under physiological pH. The coating can be selected from alginate, gellan, matrigel, cellulose derivatives, xanthan gum, HA, collagen, gelatin particle suspension, polydopamine, poly-D-lysine, poly-L-lysine.

In one embodiment said coating comprises components that influence cell attachment for example adhesion molecules, integrin ligands and derivatives thereof for example fibronectin, vitronectin, laminin, fibrillar and non-fibrillar collagen types. Other components include lectins, antibodies, antibody fragments, his-tagged proteins, lys-tagged proteins, positively charged proteins, methylated collagen and derivatives thereof. Other examples include soluble CD44 and RHAMM receptors and molecules conjugated to said receptors or antibodies against said receptors.

In a further embodiment the method of the present invention also comprises a step of washing the hydrogel after crosslinking. This is done to obtain a neutral pH, preferably a pH of about 7.

In one embodiment the hydrogel is washed at least one time in water, in a buffer having a pH of about 7 and/or in a solution comprising water and alcohol. Preferably the hydrogel is washed at least two times.

In one embodiment the method comprises a step of washing the hydrogel in water and/or in a buffer having a pH of about 7 and further comprising a step of washing the hydrogel in a solution comprising water and alcohol.

Further, pharmaceutical ingredients can be added to the HA-DVS solution to provide further properties to the hydrogel. In one embodiment the HA-DVS solution comprises at least one additional pharmaceutical ingredient such as for example a chemotherapeutic pharmaceutical ingredients. In one embodiment the pharmaceutical ingredient is selected from the group consisting of Cyclophosphamide, methotrexate, 5-fluorouracil, Doxorubicin, Mustine, vincristine, procarbazine, prednisolone, bleomycin, vinblastine, dacarbazine, etoposide, cisplatin, Epirubicin, folinic acid and oxaliplatin. In another embodiment, the pharmaceutical ingredient is a biological therapeutic.

In another embodiment the pharmaceutical ingredient is a bacteriostatic such as a bacteriocidal antibiotic. In another embodiment the pharmaceutical ingredient is selected from the group consisting of lysozymes, sulphonamides and β-lactam antibiotics.

In another embodiment the pharmaceutical ingredient is an enzyme. In another embodiment the pharmaceutical ingredient is an enzyme inhibitor such as for example hyaluronidase.

It is preferred that the hydrogel s biocompatible.

In one embodiment, the method of the present invention further comprises a step of adding living cells to said hydrogel.

The living cells can be any kind of cells such as for example cells from a vertebrate or a mammal. In a preferred embodiment said living cells are mammalian cells. The mammalian cells may for example be rodent cells such as for example mice, rat, guinea pig or hamster cells. In one embodiment the mammalian cells are human, porcine, bovine, canine, feline, and/or avian cells. In a preferred embodiment the mammalian cells are human cells. In highly preferred embodiment, the human cells are genetically modified for example by immortalization, induced pluripotency, viral gene insertion, or engineered with the CRISPR/Cas9 system.

In another embodiment the aqueous HA-DVS solution is solidified and prepared as a powder and distributed in a substrate as described. This can be done by selective laser melting (SLM).

In one embodiment the method further comprises the following steps:
- Producing a fine powder by freezing HA-DVS-ice mixture and grinding it or by freezing HA-DVS droplets.
- distribute powdery mixture evenly on a cold substrate
- selectively scanning areas of the powdery HA-DVS-ice mixture with a point heat source thereby fusing the powder
- evenly distributing a successive layer of powdery HA-DVS-ice mixture and repeating the scanning thereby building up a solid frozen HA-DVS-ice construct
- Separating unfused powdery HA-DVS-ice mixture from the solid frozen construct

Preferably, said steps are performed before contacting said construct with an alkaline solution to cross-link HA with said DVS.

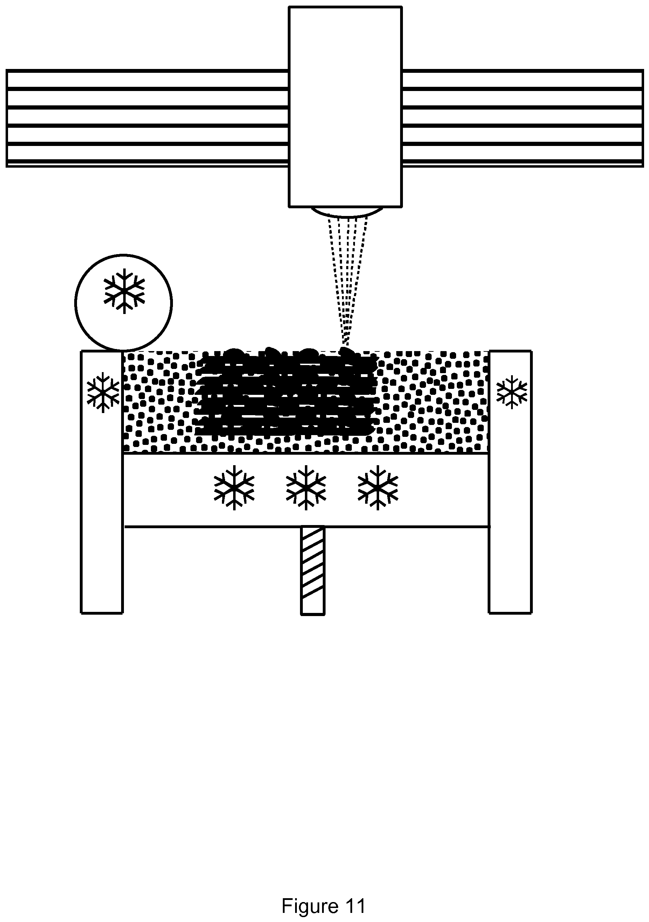

For example, in one embodiment HA, DVS, and frozen solvent can be prepared as a fine solid, powdery mixture and distributed evenly on a substrate refrigerated below the solvent melting temperature (figure 11). A collimated laser or other focused heat source will then scan a pattern on the powder to fuse the mixture in successive layers. At the end of the printing the solid printed construct is removed from the unfused powder and blown with cold dry gas. In other embodiments, a focused laser, a focused monochromatic incoherent light or a focused polychromatic light provide the energy for fusing the powdery mixture. The wavelength of the light source may preferably be set to match the absorption maximum of DVS. In a preferred embodiment, the laser is pulsed with a pulse width from 1 seconds down to 70 femtoseconds.

In yet another embodiment, a second solid component comprising frozen catalyst solution is mixed into the HA-DVS-ice powdery mixture and kept below the melting point of the first melting component. The composition of catalyst is such that it matches the melting point of the HA-DVS solution. The resulting fused solid construct will thus contain both HA-DVS and catalyst. This is advantageous as the geometry of the construct can have a solid shell.

In another preferred embodiment, said biomaterial can be mixed into the frozen powdery HA-DVS mixture to be incorporated in the scaffold.

In another preferred embodiment, a dye such as a dye pigment can be mixed into the solid powdery mixture.

In a preferred embodiment the method of the present invention occur in a controlled environment. Preferably, controlled environment means that temperature, pressure, humidity and/or gas composition is/are controlled.

In one embodiment the method further comprises a step of coating the printed construct or the hydrogel with a coating at physiological pH. Preferably, said coating os a defined herein above. Thus, the coating may for example comprise adhesion molecules, integrin ligands and derivatives thereof, lectins, antibodies, antibody fragments, polydopamine, poly-D-lysine, poly-L-lysine, his-tagged proteins, lys-tagged proteins, positively charged proteins, methylated collagen and/or thiol compounds.

In another embodiment the coating comprises enzymes and/or enzyme inhibitors and/or derivatives thereof.

### Method for producing an HA hydrogel crosslinked with BDDE

The hydrogels as described herein above can for example be crosslinked with BDDE. A method for producing a hydrogel according to the present invention and wherein HA is crosslinked with BDDE is described below.

A further aspect of the present invention relates to a method of preparing a crosslinked HA hydrogel having a predetermined pattern of ordered structural elements comprising the steps of
a. mixing HA, BDDE and a first solvent to obtain a mixture of said HA, BDDE and first solvent;
b. depositing said solution onto a cooled substrate to obtain a frozen construct;
c. lyophilizing the frozen construct to obtain a lyophilized construct;
d. immersing said lyophilized construct in a second solvent
whereby a cross-linked hydrogel is obtained.

In one embodiment HA and BDDE are mixed before addition of the first solvent. Preferably, said HA and/or BDDE is/are in the form of a powder.

The HA, BDDE and first solvent are preferably mixed by stirring as described for the HA-DVS solution.

The mixture comprising HA, BDDE and first solvent may also be referred to as the HA-BDDE solution.

The ratio of HA to BDDE may for example be in the range of from 20:1 to 1:1 by weight, such as for example from 20:1 to 2:1 by weight, or in the range of from 15:1 to 2:1 by weight, such as for example from 12:1 to 2:1 by weight, for example from 10:1 to 2:1 by weight, or in the range of from 8:1 to 2:1 by weight, such as for example from 6:1 to 2:1 by weight, for example from 5:1 to 2:1 by weight, from 4:1 to 2:1 or such as for example from 3:1 to 2:1 by weight.

In one embodiment ratio of HA to BDDE is in the range of from 10:1 to 2:1 by weight. In another embodiment the ratio of HA to BDDE may for example be in the range of from 20:1 to 3:1 by weight, such as for example from 15:1 to 3:1 by weight, such as for example from 12:1 to 3:1 by weight, for example from 10:1 to 3:1 by weight, or in the range of from 8:1 to 3:1 by weight, such as for example from 6:1 to 3:1 by weight, for example from 5:1 to 3:1 by weight, or such as for example from 4:1 to 3:1 by weight.

In a preferred embodiment the ratio of HA to BDDE is in the range of from 5:1 to 3:1.

The ratio of HA to BDDE may for example be 15:1, 10:1, 8:1, 6:1, 5:1, 4:1, 3:1 or 2:1 by weight. In a preferred embodiment the ratio of HA to BDDE is 5:1, 4:1 or 3:1 by weight.

The HA- BDDE solution may for example comprise at least 15 percentage by weight (wt%) HA and 3 wt% BDDE, such as at least 10 wt% HA and 3 wt% BDDE or such as for example at least 5 wt% HA and 3 wt% BDDE.

In another embodiment the HA- BDDE solution comprises at least 15 wt% HA and 2 wt% BDDE, such as at least 10 wt% HA and 2 wt% DVS or such as for example at least 5 wt% HA and 2 wt% BDDE.

In yet embodiment the HA- BDDE solution comprises at least 15 wt% HA and 1 wt% BDDE, such as at least 10 wt% HA and 1 wt% DVS or such as for example at least 5 wt% HA and 1 wt% BDDE.

In a preferred embodiment the HA- BDDE solution comprises 5 wt% HA and 1 wt% BDDE.

The molecular weight of HA is as defined herein above.

In one embodiment the first solvent is water or a buffer. The first solvent can for example comprise one or more salts. In one embodiment said first solvent comprises salt in a concentration of at least 100 mM. The salt concentration may preferably be as described for the solvent used in the HA-DVS solution.

In a preferred embodiment the pH of the first solvent is in the range of from 4.5 to 10, preferably from 5 to 8, or more preferably from 6.8 to 7.6.

The second solvent may comprise additional components as described above for the alkaline solution. In a preferred embodiment the pH of the second solvent is in the range of from 4.5 to 14, preferably from 5 to 8, or more preferably from 6.8 to 7.6

The HA-BDDE solution is deposited onto a cooled substrate as described above for the HA-DVS solution. Thus, the procedure for obtaining the frozen construct is the same as for the HA-DVS constructs.

However, the crosslinking procedure is different as no alkaline solution is needed to crosslink the HA-BDDE gels.

After obtaining the frozen HA-BDDE constructs, the constructs are lyophilised.

In one embodiment the second solvent is water or a buffer. The second solvent can for example comprise one or more salts. In one embodiment said second solvent comprises salt in a concentration of at least 100 mM. The salt concentration may preferably be as described above for the alkaline solution.

In one embodiment the HA-BDDE solution is deposited using at least two solutions thereby obtaining a hydrogel comprising compartments corresponding to the different solutions. For example, said at least two solutions comprise different concentrations of HA and BDDE.

The HA-BDDE solution can be printed as described above for the HA-DVS solution.

Another aspect of the present invention relates to a cross-linked HA hydrogel obtainable by the method as defined herein. Preferably, said hydrogel has a loss modulus of at least 10 Pa. It is also preferred that said hydrogel has a storage modulus greater than the loss modulus.

Yet another aspect relates to a 3D cell culture matrix comprising a hydrogel obtainable by the method as defined herein. Another aspect of the present invention relates to a wound dressing comprising the 3D cell culture matrix as defined herein. A further aspect of the present invention relates to a biosensor comprising the 3D cell culture matrix as defined herein.

It is preferred that the 3D cell culture matrix is biocompatible.

A further aspect the present invention relates to a hydrogel comprising an anisotropic stacked, fused fibrous mesh or network comprising HA and DVS, wherein the stabilized, swollen fibers have a diameter of at least 30 µm and a center to center spacing in the horizontal plane of at least 30 µm. In a preferred embodiment the swollen fibers have a diameter of 100-200 µm and a center to center spacing of 100µm to 1000 µm, also preferable is a center to center spacing of 500 µm to 2000 µm. The spacing in the vertical axis is preferably between 30 µm and 1000 µm; more preferably between 50 µm and 300 µm.

### Applications

2D cell cultures do not sufficiently take into account the natural 3D environment of cells. Therefore, 2D cell culture tests sometimes provide misleading data for *in vivo* cell responses. 3D cell culture systems may provide a better model system for the microenvironment where cells reside in tissues. Thus, the behavior of 3D-cultured cells is more reflective of *in vivo* cellular responses (Assay Drug Dev Technol. 2014 May 1; 12(4): 207-218).

Thus, another aspect of the present invention relates to use of a hydrogel prepared by the method as described herein, for making a 3D cell culture matrix. Hence, the present invention also provides a method for preparing a 3D cell culture matrix by adding living cells to the hydrogels obtained by the method as described herein above. The cell culture matrix may comprise living cells such as for example living cells as defined above. In one embodiment said 3D cell culture matrix is for use in a method of tissue repair and/or cell therapy.

Such 3D cell culture matrices can for example be useful in drug discovery, cancer cell biology, stem cell study, engineered functional tissues for implantation, and other cell-based analysis. The 3D cell culture matrices may also be useful in cytotoxicity testing, such as for example cytotoxicity testing of anticancer drugs.

Thus, one aspect of the present invention relates to use of a 3D cell culture matrix comprising a hydrogel prepared by the method as described herein, for use in drug discovery, cytotoxicity testing, stem cell research and/or cancer research and/or for engineering functional tissues. Such tissues can for example be used for implantation, tissue repair and/or wound healing
Thus, yet another aspect of the present invention relates to a 3D cell culture matrix comprising a hydrogel prepared by the method as described herein, for use in a method of cosmetic surgery and/or wound dressings.

The 3D cell culture matrix can further be used in a biosensor that can be very useful for e.g. drug screening, toxicology assays, and cell-biomaterial interaction screening, and for the identification of unknown pathogens and toxins.

Further provided is a method (not forming part of the invention) for tissue repair of an animal or a human said method comprising
- providing a hydrogel obtainable by the method as described herein
- applying said hydrogel to damaged tissue within the body of said animal or a human
whereby repair of said damaged tissue is initiated.

Tissue repair may for example include repair of bone, cartilage, wound healing, peripheral nerve damage, pelvic floor reconstruction and/or degenerative disc disease.

### Examples

### Example 1: Effects of catalyst solution and Mechanical testing

### Materials and methods

Cylindrical porous mats were printed using a pneumatic dispensing pump on a nScrypt 3dn300-TE fused deposition modelling 3D printer. The printing solution comprised 5 wt%, HA (700000 Da), 1 wt% DVS, and ddH2O. The printing dispenser was heated to 27 °C. The collector was cooled to -20 °C. The identical constructs with dimensions Ø26, h2 mm comprising 5wt% HA, 1.25 wt% DVS were printed at -25 °C and stored at -20 °C for 4-6 hours.

Three catalyst solutions were made:

| | | |
|---|---|---|
| 2 M NaOH | 3 M NaCl + 2 M NaOH | 4 M NaCl + 1 M NaOH |

with the two latter solutions having equal osmolarity and freezing point depression according to Blagden's Law. All three solutions were cooled to -10 °C, which was above the freezing point of the 2 M NaOH solution.

All constructs were immersed for 5 min in catalyst solution at -10 °C, slowly heated to 5 °C and then washed and swelled in multiple batches of room temperature PBS until pH stabilization at pH 7.4 measured with a pH meter (Radiometer, Denmark).

The degree of swelling was observed to be determined by both osmolarity and hydroxyl ion concentration:

| | | |
|---|---|---|
| 2 M NaOH: final diameter 52 mm | 3 M NaCl + 2 M NaOH: final diameter 32 mm | 4 M NaCl + 1 M NaOH: final diameter 36 mm |

The rheological measurements were carried out on a Anton Paar rheometer. The samples were measured at 25 °C. Assays were performed with plate-plate configuration and plate diameter of 25 mm. The gap was defined to 1 mm. Are performed initially a stress sweep to define resistance within the linear viscoelastic area (LVA), so the frequency sweep can be implemented with oscillation torque in the LVA. Subsequently, triplicate determination of the frequency sweep in the range of 0.1 to 10 Hz with an oscillation torque of 1.2 µNm. Wherein the elastic modulus G' and the viscous modulus G" is determined.

### Mechanical testing

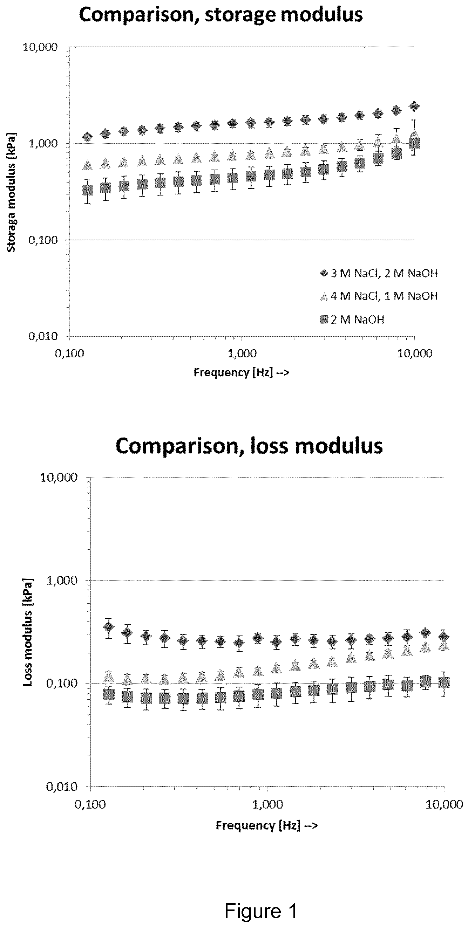

For all types of hydrogel can be seen that G'> G'', and the elastic modulus is therefore greater than the viscous modulus (Figure 1). Sample visco-elastic properties are dominated by the elastic properties, which means that the samples have a certain structure and gel strength. It may be inferred from the data that G' (3 M NaCl, 2 M NaOH) > G'(4 M NaCl, 1 M NaOH)> G '(2M NaOH).

The hydrogel properties can be altered by varying HA concentration and crosslinker ratio, and/or by varying layout (micro- and macroporosity). The results show that composition of the catalyst solution (osmolarity and OH concentration) has a profound and reproducible effect on hydrogel mechanical properties.

### Example 2: HA, DVS and lyophilization

Instead of adding catalyst solution to the printed HA-DVS construct in the hydrated, frozen state, the construct can be lyophilized and reconstituted in the catalyst solution. Ø26 mm constructs were transferred to a Labconco Triad tray freezedryer at -20 deg shelf temperature and an absolute vacuum of 0.012 Torr. The constructs were lyophilized for 96 h and subsequently kept in a desiccator at room temperature, ambient pressure for 48 h.

Reconstitution and crosslinking of the constructs were done in 3 M NaCl (aq), 2 M NaOH (aq) at -20 °C for 5 min to produce a crosslinked hydrogel. The hydrogels were then neutralized in PBS. The appearance, swelling and strength of the hydrogels were similar to scaffolds crosslinked from the frozen state from the same catalyst solution. Uncrosslinked samples can be kept for several weeks at 50% RH at room temperature without discernible macroscopic changes.

Figure 2 shows a SEM micrograph of lyophilized HA with DVS. Elongated microporosity/texture on the 200 µm wide fibers arise from thermally induced phase separation of water from Hyaluronic acid during solution quenching from 35 °C to -25 °C. The morphology of microtexture can be varied by addition of non-solvents, gases, salts or combinations thereof to the solution.

### Example 3: Reconstituting cross-linked lyophilized hydrogels

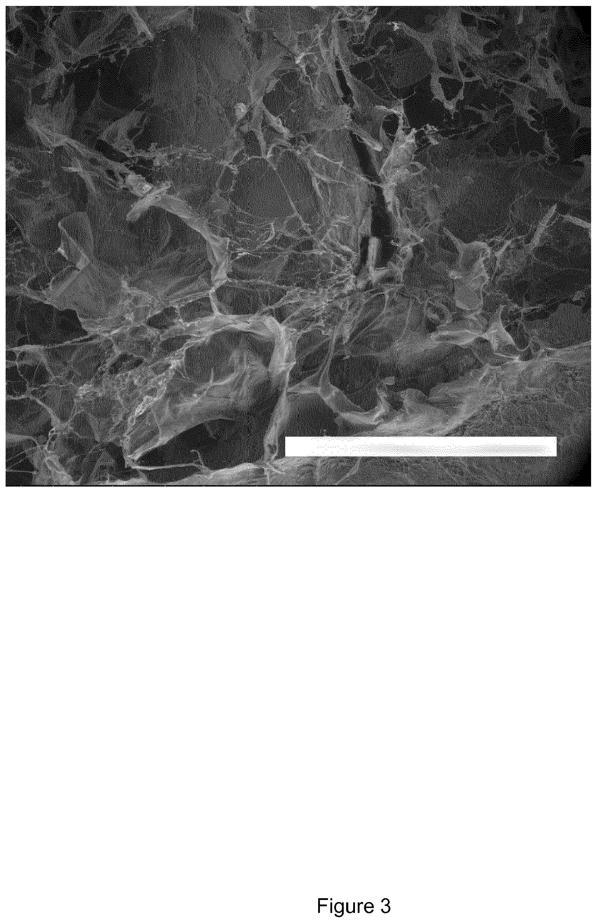

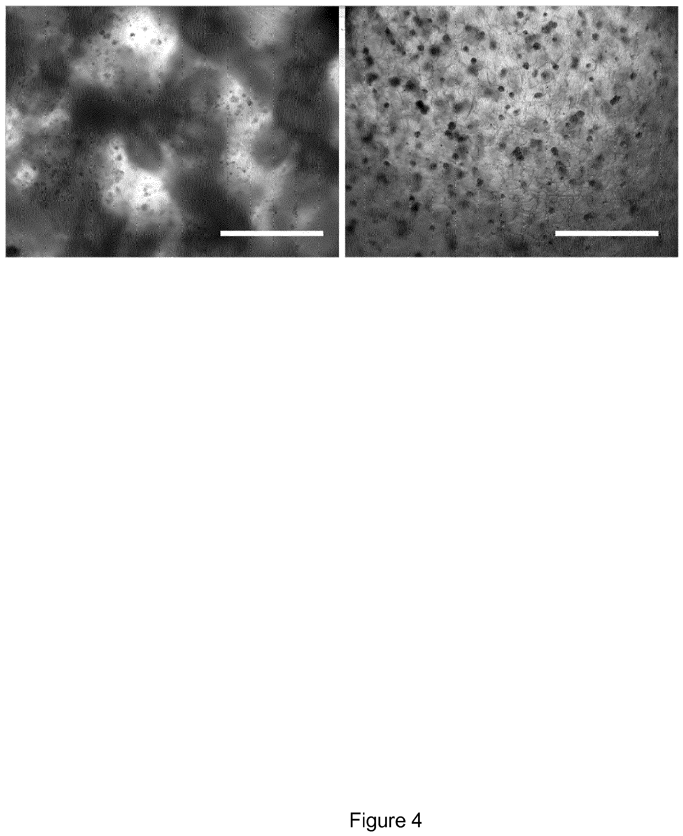

Following crosslinking in 3 M NaCl, 2 M NaOH and pH neutralization in PBS, hydrogels were washed thoroughly in ddH₂O and gently aspirated for excess water. The hydrogels were refrigerated at 5 °C for 1 h and then frozen in a -20 °C freezer for 4 h. Frozen samples were transferred to a Labconco Triad freeze-dryer and lyophilized at 0.012 T for 24 h. SEM imaging (see Figure 3) shows that the fibers in the hydrogels appear solid and macropores are spanned by strands of crosslinked Hyaluronic acid. In contrast to the hydrogels shown in Figure 4, morphological traces of ice crystal formation have been obliterated.

The dried hydrogel retained pre-freeze dimensions, were coherent and could be handled, cut, and punched with little difficulty. No salt precipitation was observed under an electron microscope. Reconstitution in PBS produces a gel with similar handling characteristics as a freshly crosslinked gel.

Should the ddH2O washing step be omitted prior to lyophilization, dried gels kept in 50% RH would initially keep dimensions but over days start to shrink to approximately 50% of the pre-freeze diameter e.g. 030mm --> 015mm. Upon reconstitution in PBS or ddH2O, dried shrunken gels would very rapidly swell to the pre-freeze size and morphology with comparable pliability and strength. In this embodiment, the hydrogel could be used as a filler or tamponade product in minimally invasive procedures.

### Example 4: In vitro 3D culture and comparison of Algimatrix™ 3D culture matrix with HA-DVS gels

An animal-free, high reproducibility 3D cell culture matrix is highly valuable for cell biology research, drug discovery and drug testing. HAluronate-DVS matrix does not directly provide integrin binding sites (e.g. RGD sequences) but it possesses moieties that are recognized by mammalian cells and their surface receptors, e.g. HAluronan-mediated motility receptor (RHAMM) and CD44. In contrast, existing animal-free cell culture matrices are based on synthetic polymers e.g. polystyrene, polylactic acid, or polycaprolactone or plant derived carbohydrates.

Following study demonstrates fabrication, preparation, and handling of a prototype 3D printed HA-DVS matrix for 3D cultivation of hepatic cells. To increase cell retention in these scaffolds, an additional embedding step was included to fill the space between fibers with a dilute, weak HA-DVS gel. Algimatrix™ 3D cell culture matrix was used as a gold standard.

### Materials and methods

### Scaffolds

3D printed frozen constructs with a diameter of 26mm, height of 2 mm were prepared as described in above examples. Frozen, uncrosslinked constructs were kept frozen at -20 °C for 4-6 hours.

30 mL 3 M NaCl + 2 M NaOH alkaline solution comprising 1% (w/v) HA was prepared by first dissolving 300 mg HA in 2.7 mL ddH₂O for 1 hour to obtain a clear, homogenous, viscous solution comprising 10 wt% HA. 5.26 g of NaCl salt was dissolved in 9 mL of ddH₂O to obtain a 36.8 wt% solution. The HA and NaCl solutions were mixed in syringes for 30 min. 2.4 g of NaOH pellets were dissolved in 15 mL of ddH₂O to obtain a 13.8 wt% NaOH solution which was as then mixed into to HA-NaCl solution and mixed in syringes for 3 minutes. The volume of the catalyst solution was adjusted with ddH2O to a final volume of 30 mL. This yielded a final concentration of 3 M NaCl + 2 M NaOH + 1 % (w/v) HA of and the solution was rapidly cooled to -20 °C before contacting the frozen constructs.

The sequence of mixing and the short duration from mixing in the NaOH solution into the HA-NaCl solution was devised to avoid excessive alkaline degradation of the HA. The 3D printed constructs were embedded with the viscous alkaline solution at -10 °C for 5 min, slowly heated to 5 °C and then washed and swelled in multiple batches of room temperature PBS until pH stabilization at pH 7.4 measured with a pH meter (Radiometer, Denmark). Hydrogels were washed thoroughly in ddH2O and gently aspirated for excess water. The final swollen diameter of 32 mm of the embedded constructs matched that of the previous example. From these hydrogels, smaller scaffolds with diameter of 6 mm, h 2 mm were punched using sterile biopsy punches and distributed in low attachment 96-well plates for refrigeration, freezing, and lyophilization according to previous examples.

Algimatrix™ 3D cell culture matrix in 96-well format was used according to manufacturer's instructions in low attachment 96-well plates.

### Cell culture

Vials of human terminally differentiated hepatic cells, HepaRG™, were thawed according to manufacturer's instructions in William's E media supplemented with HepaRG™ Thaw, Plate, & General Purpose Medium Supplement and GlutaMAX™. Scaffolds were seeded with a cell suspension volume of 30µL for a density of 300,000 cells/scaffold. Both scaffold types soaked up the suspension rapidly without saturating. Scaffolds were incubated for 1 h before adding additional cell culture media for a final volume of 200 µL. Algimatrix was seeded with same cell concentration with 10% firming buffer.

Cell culture was incubated for 7 days in a humidified incubator at 37 °C, 95% RH, 5% CO2. The entire media volume was changed each day. Samples were terminated at day 0, 1, 3, and 7 for confocal microscopy and Picogreen DNA assay.

### Picogreen DNA assay

Triplicate samples for each timepoint were transferred to Eppendorf tubes with 200 µL DMEM without Phenol Red and frozen and stored at -20 °C to lyse cells. HA-DVS samples were thawed and individually sonicated for 1 minute to desintegrate the matrix and further lyse the cells. Collagenase was added to the lysis volume incubated for 3 hr followed by addition of proteinase K and incubation at 45 °C overnight. The lysis volume was centrifuged at 10, 000 g for 5 min and used for Picogreen Assay.

To investigate any interaction between the material and the DNA assay, 300,000 cells were transferred directly to Eppendorf tubes with 200 µL DMEM without Phenol Red and a scaffold and frozen. This procedure eliminates the effects of cell seeding efficiency and retention efficiency on DNA readout.

### Confocal microscopy

Samples were gently washed in PBS and fixed in 10% neutral-buffered formalin. Cells were stained with SYTO 16 nuclear stain and Alexa Fluor® 647 Phalloidin actin stain to visualize the cell cytoskeleton and cell outline in general.

### Scanning electron microscopy

Cell-free scaffolds were lyophilized and sputter coated with gold (figure 3).

Cell culture samples were gently washed in PBS and fixed and stored in 10% neutral-buffered formalin. This was replaced with 2.5% Glutaraldehyde in 0.1 M Sodium cacodylate buffer (pH 7.4) at 4 °C overnight and dehydrated in a 50%, 70%, 96%, 99% ethanol concentration series and airdried.

Cells and scaffolds were visualized in environmental mode which obviated the need for sample coating.

### Results

### Handling

While noticeably softer, dried and hydrated HA scaffolds could be handled with same tools and procedures as Algimatrix throughout the culture period. The HA-DVS material was fully compatible with the downstream cell analysis methods.

The 3D culture could be inspected with inverted microscope throughout the culturing period. The specific arrangement of 3D printed fibers in these scaffolds obscured some areas of the 3D culture, yet the majority of the scaffold was highly transparent and revealed cell aggregates suspended in the dilute embedding gel (Figure 4). Inspection of bottom of the cell culture wells showed adherent cells immediately beneath and around the scaffolds throughout the culture period.

### DNA assay

Directly detecting and quantifying DNA are important in multiple applications. These include standard molecular biology techniques, such as synthesizing cDNA for library production and purifying DNA fragments for subcloning, as well as diagnostic techniques, such as quantifying DNA amplification products and detecting DNA molecules in drug preparations. Subsequently, it is imperative that a cell culture matrix does not interfere significantly with DNA/RNA detection or purification. Hyaluronic acid is negatively charged at physiological pH and would not have ionotropic interactions with dsDNA.

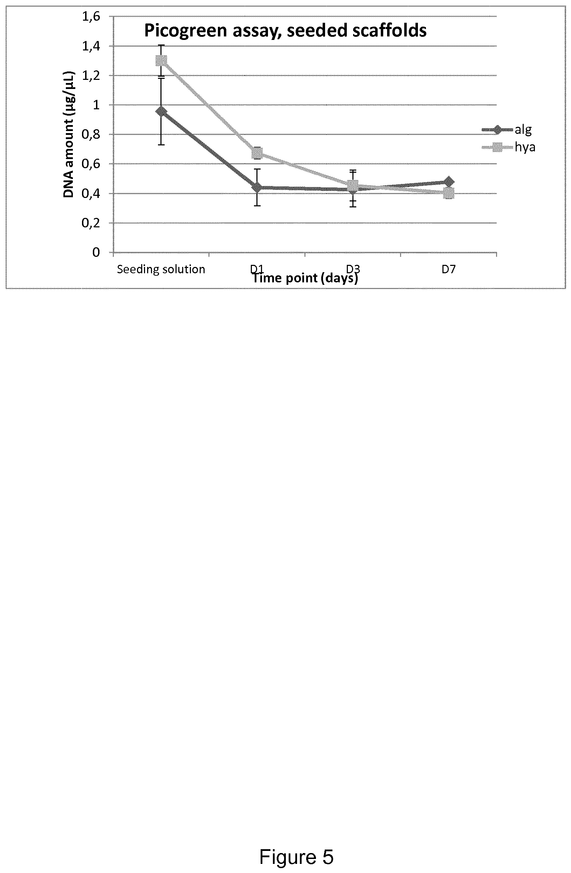

Picogreen assay on cell seeding solutions and cell seeding solution prepared with Algimatrix or HA-DVS show marked differences between DNA readout of the two matrices (Figure 5). A cell seeding solution consisting of cells and cell culture media is used as a reference for HA-DVS. There was no significant difference between DNA amounts detected from cells in solution and cells embedded in HA-DVS scaffolds. This strongly suggests that HA-DVS does not interfere with the assay. On the other hand, the presence of Algimatrix firming buffer significantly lowered the DNA readout, most likely due to ionotropic interaction between DNA and Ca2+ ions in the firming buffer. No DNA signal could be detected when cell suspension+firming buffer+ Algimatrix scaffold was assayed. Strong interaction between Ca-Alginate and DNA is thus inferred.

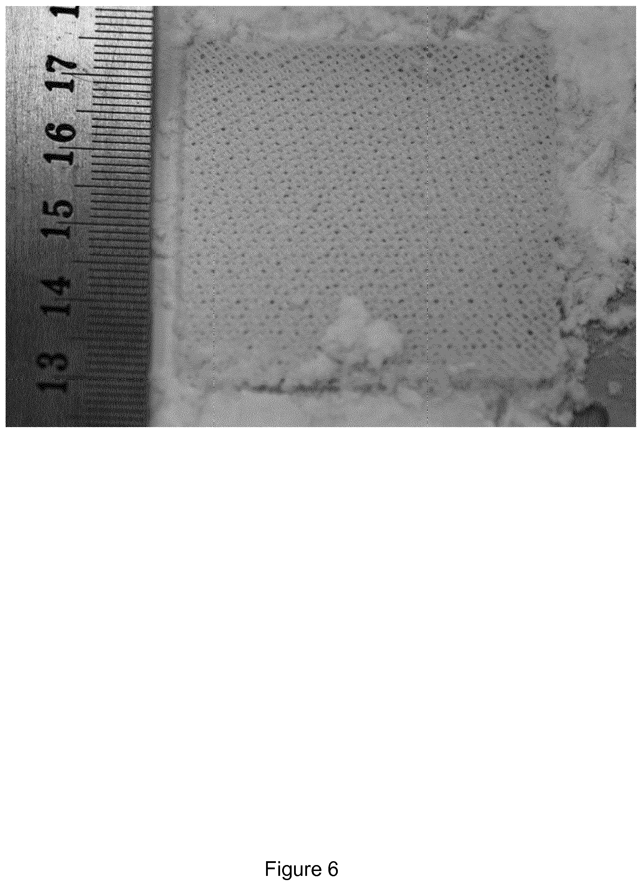

During cell culture, DNA content in HA-DVS scaffolds dropped rapidly from day 0 to day 1 with more gradual decrease for the time points, day 3 and day 7 (Figure 6). This suggests that cells are slipping out from both scaffold types during the first addition of media.

### Example 5: HA gel scaffolds comprising cells

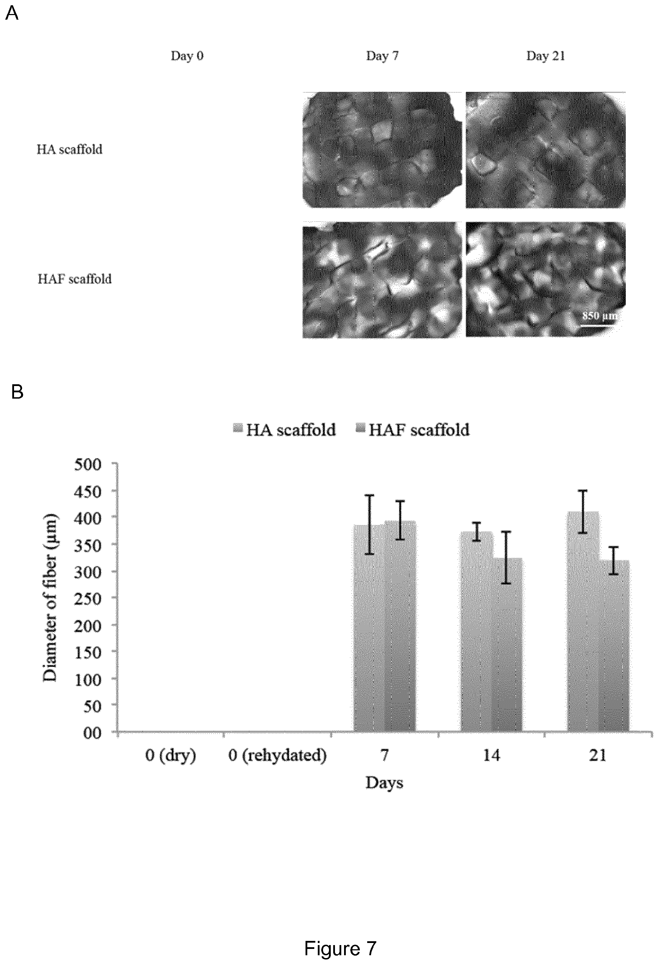

30x30x2 mm porous mats were printed using a pneumatic dispensing pump on a nScrypt 3dn300-TE fused deposition modelling 3D printer. The printing solution comprised 5 wt%, HA, 1 wt% DVS, and ddH₂O. The printing dispenser was heated to 27 °C. The collector was cooled to -20 °C. The printing area was purged with 100 % nitrogen which was feed through a dry ice cooled heat exchanger. This was to prevent water vapor to condense on the collector and printed construct. Placing sublimating dry ice around and above the printing area would also displace ambient air. Cold air purging had the extra benefit of cooling down the most recent / uppermost layer as the distance to the collector increased (Figure 7).

The mat was kept at -20 °C for 2 hours and then transferred to a 3 M NaCl, 2 M NaOH liquid catalyst solution at -20 °C while ensuring complete penetration of the frozen porous HA construct by the liquid catalyst solution. The temperature of immersed construct was slowly increased to 5 °C at a rate of 0.5 °C per minute. The pliable, cross-linked mat was then washed in copious amounts of water to stabilize the hydrogel pH at 7.4 and washed three times in PBS at 37 °C to achieve a final size of 36x36x2.5 mm.

Individual cylindrical scaffolds were punched out from the wet mat using a sterile 4 mm biopsy punch (Miltex Inc.) inside a sterile flow hood.

To investigate the effects of non-specific adsorption of fibronectin on cell attachment, a second group of scaffolds were incubated in a solution of 50 µg/mL fibronectin (Sigma-Aldrich) in PBS at 37 °C for 1 hour and then washed 3 times in PBS.

### Scaffold Morphology

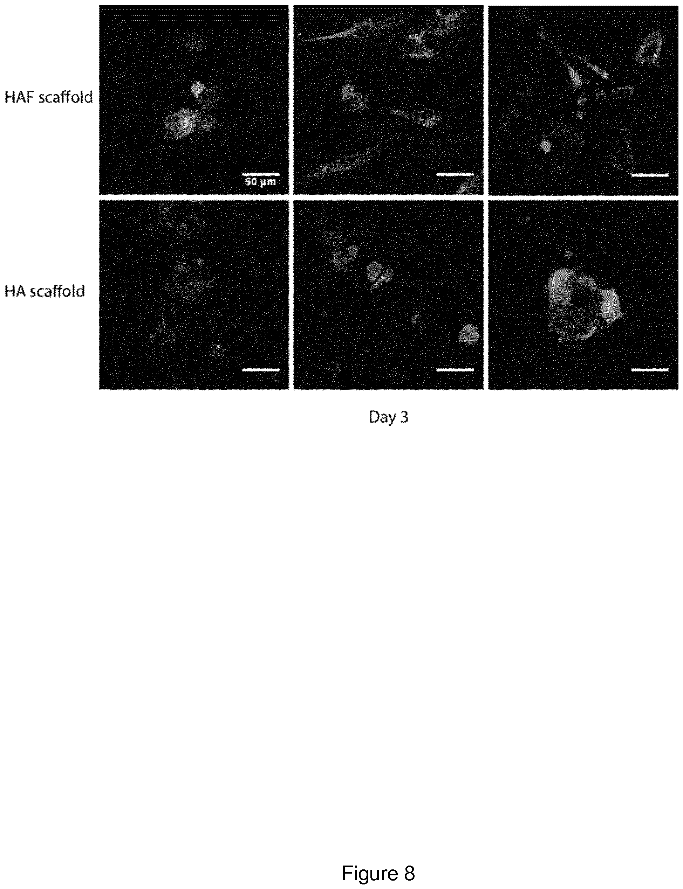

Scaffolds of the HA and HAF groups showed negligible change in fiber thickness and spacing when viewed under light microscope following 7 and 21 days in cell culture medium at at 37 °C, 100 % RH, 5% CO₂ (Figure 8)

### Cell culture

Scaffolds were seeded with 25 µL of 1,000,000 cells/ml cell suspension of human bone marrow mesenchymal stem cells (BM-MSCs) (ATCC® PCS-500-012) and were incubated at 37 °C, 100 % RH, 5% CO₂. Proliferation culture medium was DMEM, 10% fetal bovine serum, 1% GlutaMAX and 1% penicillin/streptomycin.

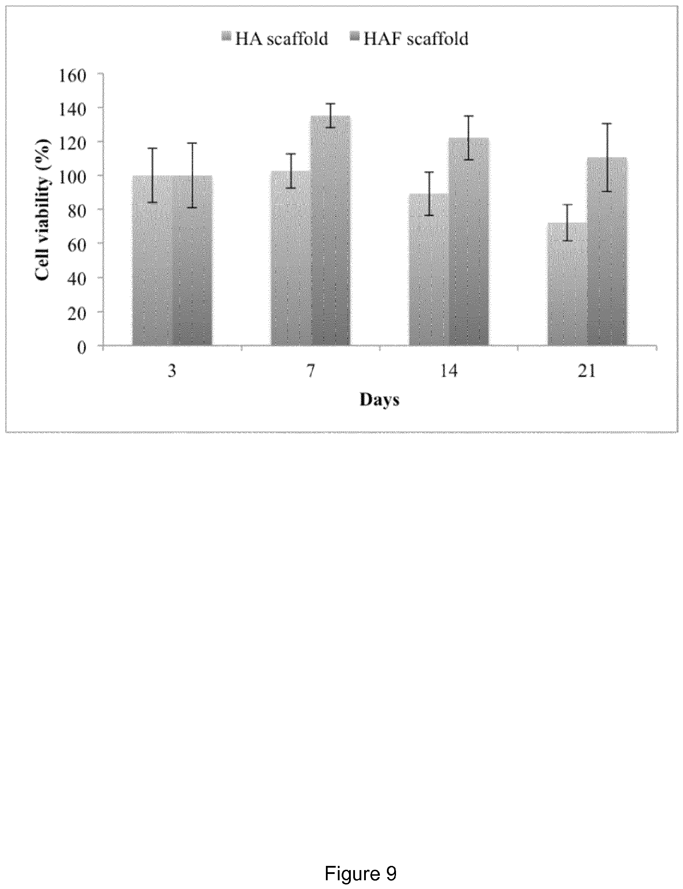

Cells were inspected with confocal microscopy on day 3 to investigate cell spreading on the scaffold fibers. Generally for Mesenchymal stem cells (MSCs), spread out, spindle shaped morphology indicated good attachment and motility on the scaffold surface. Cells were stained with Hoechst nuclear stain and Alexa Fluor 488 Phalloidin to visualize the cytoskeleton and cell outline (Figure 9).

Fibronectin coated scaffolds showed more spread out individual cells, while pure Hyaluronic acid scaffolds displayed clustered and rounded cells (Figure 9). Such arrangement is typical of cells seeded on low attachment 3D matrices.

### Progression of cell viability on HA scaffolds with and without fibronectin.

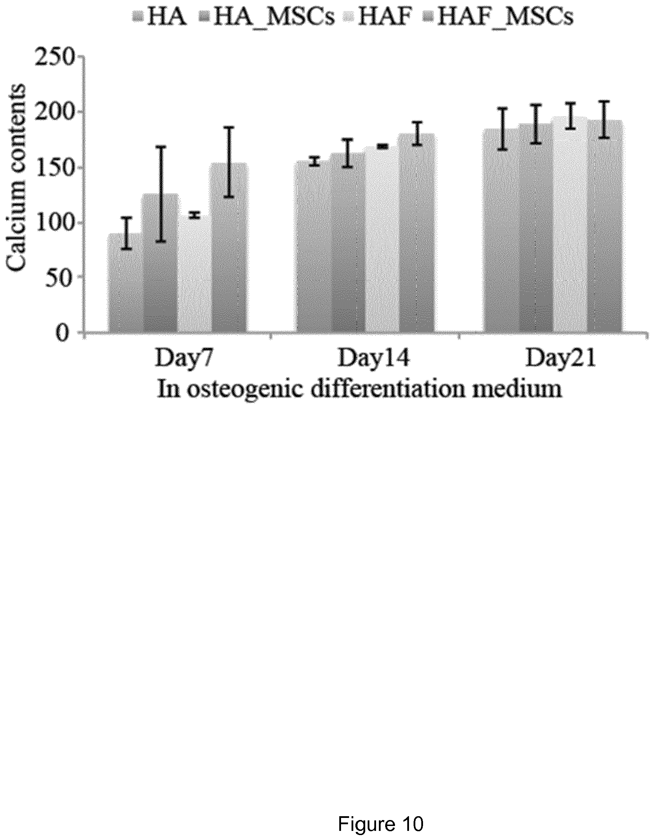

Cell viability was measured in triplicates using XTT assay (Thermo Scientific) and normalized to day 3. No proliferation was observed on printed HA scaffolds without fibronectin coating with a statistically significant decrease in viability between day 3 and 21 (Figure 10). Fibronectin treated scaffolds (HAF scaffolds) showed an increase in cell viability from day 3 to day 7 hereafter the viability was unchanged.

### Calcium deposition

HA scaffolds with and without fibronectin were tested for their ability to induce osteogenic differentiation as measured through scaffold mineralization. Hyaluronic acid itself is able to chelate calcium ions and cell-free blank controls were incubated alongside the cell cultures.

Osteogenic culture medium comprised DMEM/10% FBS for 1 week. The medium was then replaced with osteogenic stimulation media (DMEM/10% FBS with 100 nM dexamethasone, 290 IM ascorbic acid, and 5 mM b-glycerophosphate). Calcium contents were quantified by colorimetric endpoint assay (Figure 11).

The calcium assay showed that cell-free scaffolds significantly increased calcium content over 3 weeks equaling that of MSC seeded scaffolds. Scaffolds with MSCs, however, showed accelerated uptake.

### Example 6: HA-DVS hydrogels comprising particles

A 5wt% HA-DVS solution was made with pH adjusted to 8.5 to decrease viscosity. HA solution was extruded out of a 27G tapered needle to form a droplet which was then sequentially deposited to a collecting substrate cooled to -17 °C. The HA-DVS solution in the printing needle was 31 °C to ensure melting and subsequent fusion of the material to underlying layers. The dot spacing was 0.300 mm and layer height was 0.200 mm. Following printing, the construct was lyophilized and gold coated in preparation for scanning electron microscopy (SEM). Visualization of hydrated or lyophilized hydrogels using photography or SEM, respectively, was made difficult by the high transparency when hydrated or the collapse of the shape when lyophilized.

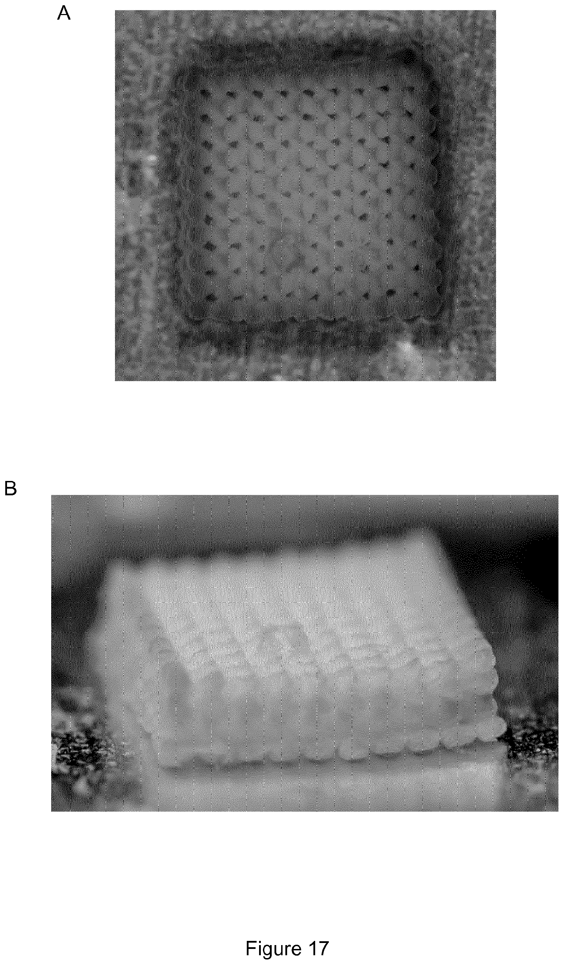

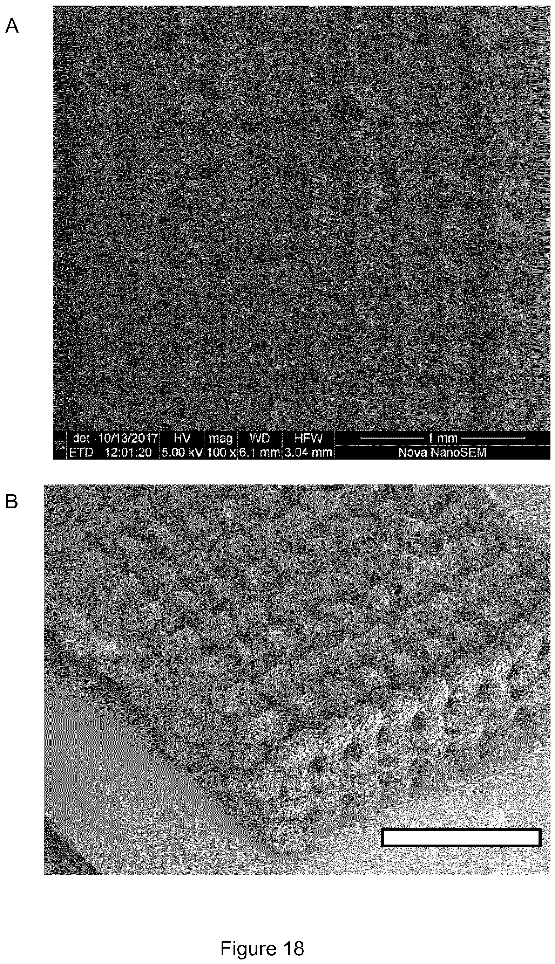

HA-DVS constructs comprising particles are shown in Figures 17 and 18.

### Example 7: HA gels comprising tyramine as a crosslinker

Scaffolds with a diameter of 4 mm and height of 2 mm were seeded with 25 µL of 1,000,000 cells/ml cell suspension of HepaRG liver cell line (Life Technologies). Scaffolds were incubated at 37 °C, 100 % RH, 5% CO₂. Culture medium was DMEM, 10% fetal bovine serum, 1% GlutaMAX and 1% penicillin/streptomycin.

Cell viability was measured in triplicates using XTT assay (Thermo Scientific) and normalized to day 3. Cell viability increased with 22 % on day 7.

### Printing tyramine modified HA scaffolds

HA with a 5% tyramine substitution was mixed with PBS buffer containing Horseradish Peroxidase (10 U/mL) for a 5 wt% HA solution. Printing was done similarly to that of HA-DVS. Printed, un-crosslinked scaffolds were kept at -20 °C for 2 hours and then immersed in a 200 mL bath of 5% H₂O₂, 0.5 M NaCl at a temperature of -10 °C. The temperature was slowly increased by placing the bath in a 5 °C refrigerator. After 30 mins, the temperature of the bath was 5 °C and the scaffold was crosslinked and pliable with sufficient strength to be handled albeit much softer than the above embodiment of HA-DVS .

Whereas a skilled person will likely alter hydrogel properties by varying composition and concentration of components such as for example HA and/or crosslinker, and by varying layout, such as macroporosity, it is now shown that composition of the catalyst solution (osmolarity and OH concentration) has a profound and reproducible effect on hydrogel mechanical properties.

### Example 8: HA gels comprising BDDE as a crosslinker

### Embedding BDDE 3D constructs with a secondary HA matrix

Frozen 5% HA-BDDE 4:1 constructs with a porosity of 80 % were kept at a temperature of -3.0 °C on a cryostat. A freezing point depressed 1wt% HA-BDDE 10:1 aqueous solution comprising 0.150 M NaCl was embedded in the frozen construct. Following 5 mins at -3 °C, the embedded construct was cooled to -20 °C and lyophilized for 48 hours at shelf temperature of -20 °C, freeze trap temperature of -80 °C, and vacuum level of 0.012 Torr.

Reconstitution in ddH2O at 5 °C produced a crosslinked hydrogel comprising the opaque 3D printed network embedded with a weaker, more transparent hydrogel. The gels were washed five times in ddH2O and punched out into smaller scaffolds which were lyophilized.

In a variation, frozen 3D printed 5% HA-BDDE 4:1 constructs with a height of 3 mm, porosity of 80 % were kept at a temperature of -20 °C on a cold plate. The constructs were blown with cold, dry air to prevent water vapor condensation on the construct surfaces. A 1 wt % HA-BDDE 10:1 aqueous solution was heated to 10 °C and rapidly filled homogenously into the pores of the 3D printed construct whereafter the 1%wt solution froze within seconds.

The embedded frozen constructs were lyophilized for 48 hours at shelf temperature of - 20 °C, freeze trap temperature of -80 °C, and vacuum level of 0.012 Torr. Scanning electron microscopy of the lyophilized embedded construct revealed a fine porous foam with pores sizes down to 1 µm embedded in a denser 3D printed mesh.

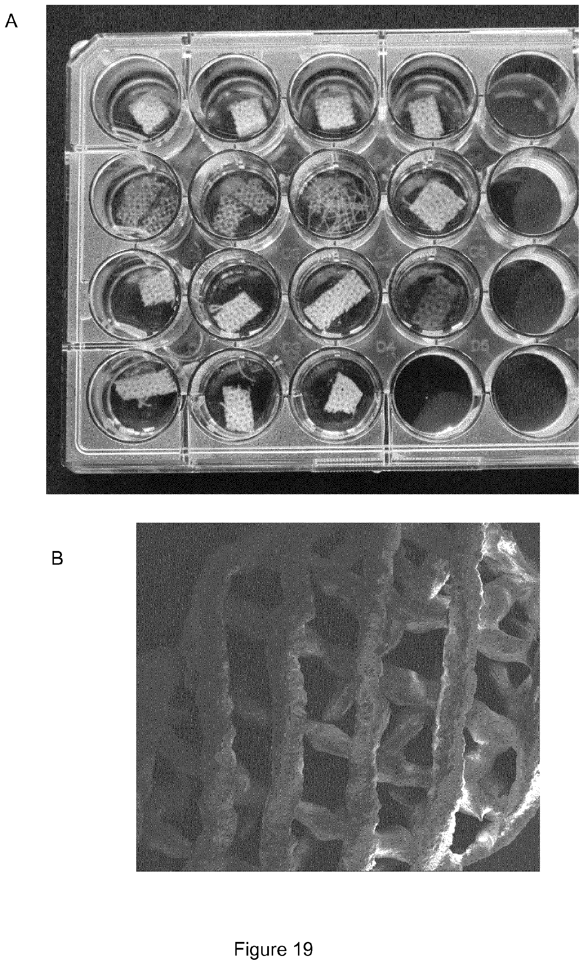

Reconstitution with ddH2O produced a crosslinked hydrogel comprising the opaque 3D printed network embedded with a weaker, more transparent hydrogel. Figure 19 shows SEM imaging of the HA-BDDE scaffold illustrating that using BDDE as a crosslinking agent can produce the same structures as when using DVS as a crosslinking agent.

### Embedding HA-BDDE 3D constructs with gelatine

A significant benefit of crosslinking with BDDE at physiological pH and molarity is possibilities for functionalization with sensitive macromolecules, e.g. polypeptides and proteins, without these precipitating from solution, denaturing, or degrading.

In this example, frozen 3D printed 5% HA-BDDE 4:1 constructs with a height of 3 mm, porosity of 80% were kept at a temperature of -30 °C on a cryostat. A 0.5 wt% porcine gelatine aqueous solution was kept at 20 °C in square thin-walled PTFE mould with side lengths of 42 mm size and with the depth of the solution at 3 mm. The frozen construct was dipped for 1 second in the gelatine solution and then set back the cryostat with following lyophilisation. The reconstituted HA-gelatine hydrogel was washed 5 times in ddH2O for 30 minutes each on orbital shaker. We used a 0.5 % wt genipin solution to stain and detect the presence of gelatine remaining on the HA hydrogel 3D structure. Genipin reacts with primary amine groups on lysine residues in the gelatine to form a blue product.

### Stacked, sequential 3D printing with FDM

In some production scenarios, it is advantageous and cost-efficient to produce the maximum amount of product between necessary operator attendance. For fused deposition modelling, limitations in available substrate area i.e. printing space require operator attendance or robotic automation to clear the finished frozen 3D construct from the printing substrate in preparation for the next printing run. To mitigate the need for operator attendance we devised a method in which the first, flat, frozen construct is laid down as described in previous examples. Following, while the substrate temperature remained unchanged, printing was paused and water vapour was allowed to condense on the construct for approximately 3 minutes to form a thin layer of condensate on the top surface. When the printing of the overlying construct commenced, its first layer connected and fused to a porous layer of ice which had sufficient shear strength to keep the overlying construct in place during printing. Stack heights of approximately 7 mm, i.e. at least three 2 mm constructs, were possible without the need for further forced air cooling of the constructs. Forced air cooling allowed higher stacks.

After the construct stack was finished, the individual constructs could be pried apart and crosslinked or lyophilized separately. Alternatively, the stack could be lyophilized whole, which removed the connecting ice layers to separate the individual lyophilized constructs.

### Example 9: Embryoid bodies cultured in HA-DVS scaffolds

Scaffolds were non-covalently coated with laminin through incubation with 10 µg/mL laminin + fibronectin in PBS at 37 °C overnight. Following washing in PBS, scaffolds were freeze dried. The human embryonic stem cell line H9 was cultured on a feeder-free system with E-8 media prior to the experiment. Cells were detached from the plate and suspended in fresh E-8 media with added 10µM Y-27632. Y-27632 is a cell permeable inhibitor of Rho-associated, coiled-coil containing protein kinase (ROCK). Cells were seeded on the dried, coated scaffold with cell density of 300,000 cells/scaffold. After 2 hours, N2B27 neural media was added to the seeded scaffolds. For 4 days, scaffolds were maintained with N2B27 + glycogen synthase kinase-3 inhibitors, SB and CHIR, and Smoothened Agonist. Media composition was then changed to SAG and FGF for 1 week. At day 11, neural maturation media was used until termination on day 32. Intact cell aggregates were easily released from the scaffold by tearing the porous hydrogel with forceps. Immunostaining showed double positive for FOXA2 transcription factor and Tyrosine hydroxylase, which are markers of dopaminergic neurons.

In summary, dopaminergic neuron formation from embryonic cells was supported in a HA-DVS scaffold coated with attachment proteins laminin and fibronectin.

## Claims

1. A hydrogel comprising multiple layers and a porous structure of ordered structural elements, wherein said structural elements are fibers and/or particles where
a) said fibres have a diameter in the range of from 50 µm to 1000 µm, and wherein said hydrogel comprises multiple layers and wherein fibres between neighbouring layers are not parallel and where at least one layer of said hydrogel consists of or comprises alternating coiled and straight fibres, and/or
b) said particles have a diameter in the range of from 30 µm to 1 mm and wherein said particles are aligned in rows
wherein said structural elements comprises hyaluronic acid crosslinked with DVS, BDDE and/or tyramine.

2. The hydrogel according to claim 1, wherein each of said coiled fibres forms a periodic curve.

3. A method of preparing a crosslinked HA hydrogel having a predetermined pattern of ordered structural elements, said method comprising the steps of
a. mixing HA, DVS and solvent to obtain a mixture of said DVS, HA and solvent wherein said solvent has a pH below 9;
b. depositing said solution onto a cooled substrate to obtain a frozen construct;
c. contacting (or immersing) said frozen construct comprising HA, DVS and solvent, which has not yet been crosslinked, with an alkaline solution having a pH above 10 at a temperature below the freezing point of the construct to cross-link HA with DVS,
whereby a cross-linked hydrogel is obtained.

4. The method according to claim 3, wherein said alkaline solution comprises at least one drug carrier.

5. The method according to any one of claims 3-4, wherein the solution is deposited using at least two solutions thereby obtaining a hydrogel comprising compartments corresponding to the different solutions.

6. A method of preparing a crosslinked HA hydrogel having a predetermined pattern of ordered structural elements, said method comprising the steps of
a. mixing HA, BDDE and a first solvent to obtain a mixture of said HA, BDDE and first solvent;
b. depositing said solution onto a cooled substrate to obtain a frozen construct;
c. lyophilizing the frozen construct to obtain a lyophilized construct;
d. immersing said lyophilized construct in a second solvent
whereby a cross-linked hydrogel is obtained.

7. The method according to claim 6, wherein said HA and BDDE are mixed before addition of the first solvent.

8. The method according to any of claims 6-7, wherein said frozen construct is embedded in a second solution comprising HA before lyophilizing said frozen construct.

9. The method according to any one of claims 6-8, wherein said solution is deposited using a device comprising a motor-driven nozzle.

10. The method according to any one of claims 6-9, wherein said solution is deposited using fused deposition modelling (FDM).

11. The method according to any one of claims 3-10, wherein said solution is deposited in a layer-by-layer process thereby obtaining a construct comprising one or more layers.

12. Use of a hydrogel prepared by the method according to any one of claims 3-11 or the hydrogel according to any one of claims 1-2, for making a 3D cell culture matrix.

13. The use according to claim 12, wherein said 3D cell culture matrix is for use in a method of tissue repair and/or cell therapy.

14. Use of a hydrogel prepared by the method according to any one of claims 3-11 or the hydrogel according to any one of claims 1-2, for drug development, stem cell research and/or cancer research, or the hydrogel prepared by the method according to any one of claims 3-11 or the hydrogel according to any of claims 1-2, for use in a method of cosmetic surgery or for use as wound dressing in a method of dressing wounds.

15. A 3D cell culture matrix comprising the hydrogel according to any one of claims 1-2.

## Patentansprüche

1. Hydrogel, das mehrere Schichten und eine poröse Struktur aus geordneten strukturellen Elementen umfasst, wobei die strukturellen Elemente Fasern und/oder Partikel sind, wobei
a) die Fasern einen Durchmesser in dem Bereich von 50 µm bis 1000 µm aufweisen, und wobei das Hydrogel mehrere Schichten umfasst, und wobei Fasern zwischen benachbarten Schichten nicht parallel sind, und wobei mindestens eine Schicht des Hydrogels aus sich abwechselnden gewickelten und geraden Fasern besteht, und/oder
b) die Partikel einen Durchmesser in dem Bereich von 30 µm bis 1 mm aufweisen, und wobei die Partikel in Reihen ausgerichtet sind
wobei die strukturellen Elemente Hyaluronsäure, die mit DVS,BDDE und/oder Tyramin quervernetzt ist, umfasst.

2. Hydrogel nach Anspruch 1, wobei jede der gewickelten Fasern eine periodische Kurve bildet.

3. Verfahren zum Herstellen eines quervernetzten HS-Hydrogels, das ein vorbestimmtes Muster aus geordneten strukturellen Elementen aufweist, wobei das Verfahren die folgenden Schritte umfasst:
a. Mischen von HS, DVS und einem Lösungsmittel, um ein Gemisch aus dem DVS, der HS und dem Lösungsmittel zu erhalten, wobei das Lösungsmittel einen pH-Wert unterhalb von 9 aufweist;
b. Anordnen der Lösung auf einem gekühlten Substrat, um ein gefrorenes Konstrukt zu erhalten;
c. Inkontaktbringen des gefrorenen Konstrukts, das HS, DVS und das Lösungsmittel umfasst, das noch nicht quervernetzt worden ist, mit einer alkalischen Lösung, die einen pH-Wert oberhalb von 10 aufweist, bei einer Temperatur unterhalb des Gefrierpunkts des Konstrukts, um HS mit DVS querzuvernetzen (oder Eintauchen in diese),
wodurch ein quervernetztes Hydrogel erhalten wird.

4. Verfahren nach Anspruch 3, wobei die alkalische Lösung mindestens einen Arzneimittelträger umfasst.

5. Verfahren nach einem der Ansprüche 3-4, wobei die Lösung unter Verwendung von mindestens zwei Lösungen abgelagert wird, wodurch ein Hydrogel erhalten wird, das Kammern umfasst, die den verschiedenen Lösungen entsprechen.

6. Verfahren zum Herstellen eines quervernetzten HS-Hydrogels, das ein vorbestimmtes Muster aus geordneten strukturellen Elementen aufweist, wobei das Verfahren die folgenden Schritte umfasst:
a. Mischen von HS, BDDE und einem ersten Lösungsmittel, um ein Gemisch aus der HS, dem BDDE und dem ersten Lösungsmittel zu erhalten;
b. Ablagern der Lösung auf einem gekühlten Substrat, um ein gefrorenes Konstrukt zu erhalten;
c. Lyophilisieren des gefrorenen Konstrukts, um ein lyophilisiertes Konstrukt zu erhalten;
d. Eintauchen des lyophilisierten Konstrukts in eine zweite Lösung
wobei ein quervernetztes Hydrogel erhalten wird.

7. Verfahren nach Anspruch 6, wobei die HS und der BDDE vor dem Hinzufügen des ersten Lösungsmittels gemischt werden.

8. Verfahren nach einem der Ansprüche 6-7, wobei das gefrorene Konstrukt vor dem Lyophilisieren des gefrorenen Konstrukts in eine zweite Lösung eingebettet ist, die HS umfasst.

9. Verfahren nach einem der Ansprüche 6-8, wobei die Lösung unter Verwendung einer Vorrichtung abgelagert wird, die eine motorisch angetriebene Düse umfasst.

10. Verfahren nach einem der Ansprüche 6-9, wobei die Lösung unter Verwendung von Fused Deposition Modeling (FDM) abgelagert wird.

11. Verfahren nach einem der Ansprüche 3-10, wobei die Lösung in einem Schicht-für-Schicht-Prozess abgelagert wird, wodurch ein Konstrukt erhalten wird, das eine oder mehrere Schichten umfasst.

12. Verwendung eines Hydrogels, das durch das Verfahren nach einem der Ansprüche 3-11 hergestellt wird oder des Hydrogels nach einem der Ansprüche 1-2 zum Fertigen einer 3D-Zellkulturmatrix.

13. Verwendung nach Anspruch 12, wobei die 3D-Zellkulturmatrix in einem Gewebereparaturverfahren und/oder einer Zelltherapie verwendet wird.

14. Verwendung eines Hydrogels, das durch das Verfahren nach einem der Ansprüche 3-11 hergestellt ist oder des Hydrogels nach einem der Ansprüche 1-2 zur Arzneimittelentwicklung, Stammzellforschung und/oder Krebsforschung oder des Hydrogels, das durch das Verfahren nach einem der Ansprüche 3-11 hergestellt ist oder des Hydrogels nach einem der Ansprüche 1-2 zur Verwendung in einem Schönheitschirurgieverfahren oder zur Verwendung als ein Wundverband in einem Verfahren zum Verbinden von Wunden.

15. 3D-Zellkulturmatrix, die das Hydrogel nach einem der Ansprüche 1-2 umfasst.

## Revendications

1. Hydrogel comprenant plusieurs couches et une structure poreuse d'éléments structurels ordonnés, dans lequel lesdits éléments structurels sont des fibres et/ou des particules où
a) lesdites fibres ont un diamètre compris entre 50 µm et 1 000 µm, et dans lequel ledit hydrogel comprend plusieurs couches et dans lequel les fibres entre les couches voisines ne sont pas parallèles et où au moins une couche dudit hydrogel est composée de ou comprend une alternance de fibres enroulées et droites, et/ou
b) lesdites particules ont un diamètre compris entre 30 µm et 1 mm et dans lequel lesdites particules sont alignées en rangées
dans lequel lesdits éléments structurels comprennent un acide hyaluronique réticulé avec du DVS, du BDDE et/ou de la tyramine.

2. Hydrogel selon la revendication 1, dans lequel chacune desdites fibres enroulées forme une courbe périodique.

3. Procédé de préparation d'un hydrogel HA réticulé ayant un motif prédéterminé d'éléments structurels ordonnés, ledit procédé comprenant les étapes
a. de mélange de HA, de DVS et d'un solvant pour obtenir un mélange desdits DVS, HA et solvant dans lequel ledit solvant a un pH inférieur à 9 ;
b. de dépôt de ladite solution sur un substrat refroidi pour obtenir une construction congelée :
c. de mise en contact (ou d'immersion) de ladite construction congelée comprenant du HA, du DVS et un solvant, qui n'a pas encore été réticulé, avec une solution alcaline ayant un pH supérieur à 10 à une température inférieur au point de congélation de la construction pour réticuler le HA avec du DVS,
moyennant quoi un hydrogel réticulé est obtenu.

4. Procédé selon la revendication 3, dans lequel ladite solution alcaline comprend au moins un vecteur de médicament.

5. Procédé selon l'une quelconque des revendications 3 et 4, dans lequel la solution est déposée à l'aide d'au moins deux solutions permettant ainsi d'obtenir un hydrogel comprenant des compartiments correspondant aux différentes solutions.

6. Procédé de préparation d'un hydrogel HA réticulé ayant un motif prédéterminé d'éléments structurels ordonnés, ledit procédé comprenant les étapes
a. de mélange de HA, de BDDE et d'un premier solvant pour obtenir un mélange desdits HA, BDDE et premier solvant ;
b. de dépôt de ladite solution sur un substrat refroidi pour obtenir une construction congelée :
c. de lyophilisation de la construction congelée pour obtenir une construction lyophilisée ;
d. d'immersion de ladite construction lyophilisée dans un second solvant
moyennant quoi un hydrogel réticulé est obtenu.

7. Procédé selon la revendication 6, dans lequel lesdits HA et BDDE sont mélangés avant l'ajout du premier solvant.

8. Procédé selon l'une quelconque des revendications 6 et 7, dans lequel ladite construction congelée est incorporée dans une seconde solution comprenant du HA avant la lyophilisation de ladite construction congelée.

9. Procédé selon l'une quelconque des revendications 6 à 8, dans lequel ladite solution est déposée à l'aide d'un dispositif comprenant une buse entraînée par un moteur.

10. Procédé selon l'une quelconque des revendications 6 à 9, dans lequel ladite solution est déposée à l'aide d'une modélisation par dépôt en fusion (FDM).

11. Procédé selon l'une quelconque des revendications 3 à 10, dans lequel ladite solution est déposée dans un processus couche par couche permettant ainsi d'obtenir une construction comprenant une ou plusieurs couches.

12. Utilisation d'un hydrogel préparé par le procédé selon l'une quelconque des revendications 3 à 11 ou hydrogel selon l'une quelconque des revendications 1 et 2, pour fabriquer une matrice de culture cellulaire 3D.

13. Utilisation selon la revendication 12, dans laquelle ladite matrice de culture cellulaire 3D est destinée à être utilisée dans un procédé de réparation tissulaire et/ou de thérapie cellulaire.

14. Utilisation d'un hydrogel préparé par le procédé selon l'une quelconque des revendications 3 à 11 ou hydrogel selon l'une quelconque des revendications 1 et 2, pour le développement de médicaments, la recherche sur les cellules souches et/ou la recherche sur le cancer ou hydrogel préparé par le procédé selon l'une quelconque des revendications 3 à 11 ou hydrogel selon l'une quelconque des revendications 1 et 2, destiné à être utilisé dans un procédé de chirurgie esthétique ou pour une utilisation comme pansement dans un procédé de traitement des plaies.

15. Matrice de culture cellulaire 3D comprenant l'hydrogel selon l'une quelconque des revendications 1 et 2.
